(19) Europäisches Patentamt / European Patent Office / Office européen des brevets

(11) **EP 4 129 485 A1**

(12) **EUROPEAN PATENT APPLICATION**

(43) Date of publication:
**08.02.2023 Bulletin 2023/06**

(21) Application number: **22191524.2**

(22) Date of filing: **10.08.2017**

(51) International Patent Classification (IPC):
**B01L 99/00** (2010.01)    **G01N 33/48** (1980.01)
**G01N 33/487** (1985.01)    **G01N 33/49** (1985.01)
**B01L 3/00** (1968.09)

(52) Cooperative Patent Classification (CPC):
**B01L 3/54; B01L 3/5027; G01N 33/49;**
B01L 2300/021; B01L 2300/022; B01L 2300/0663;
B01L 2300/0681; B01L 2300/0825;
B01L 2400/0406; G01N 2021/6439

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.08.2016 US 201662373755 P
27.09.2016 US 201662400179 P
27.09.2016 US 201662400184 P**

(62) Document number(s) of the earlier application(s) in
accordance with Art. 76 EPC:
**17840287.1 / 3 496 860**

(71) Applicant: **SRI International
Menlo Park, CA 94025 (US)**

(72) Inventors:
• **COOPER, David, E.
Palo Alto, 94306 (US)**

• **BACHER, Rowena
Scotts Valley, 95066 (US)**
• **MENDOZA, Estevan
Mountain View, 94041 (US)**
• **BALOG, Robert
Sunnyvale, 94086 (US)**
• **TODD, Kathryn
Menlo Park (US)**
• **BRADLEY, Kirk, A.
Menlo Park (US)**

(74) Representative: **D Young & Co LLP
120 Holborn
London EC1N 2DY (GB)**

Remarks:
This application was filed on 22-08-2022 as a
divisional application to the application mentioned
under INID code 62.

(54) **BIOLOGICAL SAMPLE-ANALYZING SYSTEM, COMPONENTS, AND METHODS THEREOF**

(57) Provided herein is an analyzing system including components and methods thereof. The analyzing system includes, in some embodiments, a test cartridge and an analyzer. The test cartridge can include a port for a biological sample; one or more lateral flow assay strips with one or more target capture zones for binding one or more targets of the biological sample; and a memory device including a development time for the test cartridge. The analyzer can include an opening for the cartridge; a reader/writer device for writing an initial time stamp for the biological sample to the memory device and reading it back; a processor configured to execute an algorithm and logic to ensure an elapsed time from the initial time stamp meets or exceeds the development time for the test cartridge; and a detector for detecting emissions from an up-converting phosphor in the one or more target capture zones.

FIG. 1

EP 4 129 485 A1

**Description**

CROSS-REFERENCE

This application claims the benefit and priority to under 35 USC 119 of U.S. Provisional Patent Application No. 62/373,755, filed August 11, 2016, titled "BLOOD PROCESSING DEVICE"; U.S. Provisional Patent Application No. 62/400,176, filed September 27, 2016, titled "POINT OF CARE ANALYSIS SYSTEM"; and U.S. Provisional Patent Application No. 62/400,184, filed September 27, 2016, titled "SAMPLE COLLECTING DEVICE," each of which is hereby incorporated herein by reference in its entirety.

GOVERNMENT LICENSE RIGHTS

This invention was made with government support under contract number HHSO100201000007C awarded by the Biomedical Advanced Research and Development Authority. The government has certain rights in this invention.

FIELD

This disclosure relates to biological sample-analyzing systems and, more specifically, point-of-care analysis systems with up-converting phosphor-reporter technology ("UPT"). Such systems can include devices for collecting biological samples and cartridges for preparing the biological samples for analysis.

BACKGROUND

There is a need for a biological sample-analyzing system for measurement of proteins including prions, as well as other entities such as, but not limited to, spores, bacteria, viruses, satellites, and viroids, which measurement is valuable in many situations. Examples of such situations include during an unexpected exposure to radiation (e.g., ionizing radiation) or some other harmful chemical or biological agent, or during a spread of an epidemic. Other examples include environments that are removed from a hospital or a clinic such as a pharmacy, a home, a remote village, or a town. Yet other examples include the area of veterinary medicine, as well as the areas of botanical and agricultural sampling. Need for such a system exists in many industrial operations such as, but not limited to, chemical plants, water management centers, and sewage treatment centers. Thus, there are numerous situations in need of such a system that analyzes biological samples. Provided herein are systems and methods that meet or exceed the foregoing need.

SUMMARY

An analyzing system including, in some embodiments, a test cartridge and an analyzer is discussed. The test cartridge can include a port configured to accept a biological sample, one or more test strips, and a memory device. Each test strip can include one or more target capture zones configured to bind one or more targets of the biological sample. The memory device can include information for determining a development time for the one or more test strips. The analyzer can include an opening configured to accept the test cartridge, a reader/writer, a processor, one or more sources of electromagnetic radiation, and a detector. The reader/writer can be configured to i) write to the memory device an initial time stamp for the biological sample and ii) read back from the memory device the initial time stamp for the biological sample at least when a calculation of an elapsed time occurs. The processor can be configured to invoke i) a stored algorithm configured to calculate an elapsed time from an instant time and the initial time stamp and ii) stored logic configured to ensure the elapsed time meets or exceeds the development time for the one or more test strips. The one or more sources of electromagnetic radiation can be configured to excite an up-converting phosphor present in the one or more target capture zones of the one or more test strips. The detector can be configured to detect up-converting phosphor emissions from the one or more target capture zones.

Also provided herein is an analyzing system including, in some embodiments, a test cartridge and an analyzer. The test cartridge can include a port configured to accept a biological sample, one or more test strips, and a memory device. Each test strip can include one or more target capture zones configured to bind one or more targets of the biological sample. The memory device can include a first batch of information selected from a group consisting of i) information for identifying one or more assays of the one or more test strips, ii) information for determining a development time for the one or more test strips, iii) information for determining concentration of the one or more targets of the biological sample, iv) information for interpreting assay results for the one or more targets (e.g., whether concentration is high or low, problematic or not, etc.), and v) any combination thereof. The analyzer can include an opening configured to accept the test cartridge, a reader/writer, a processor, one or more sources of electromagnetic radiation, and a detector. The reader/writer can be configured to i) read the first batch of information from the memory device, ii) write a second batch of information to the memory device including an initial time stamp for the biological sample, and iii) read back the initial time stamp for the biological sample. The processor can be configured to invoke i) a stored algorithm configured to calculate an elapsed time from an instant time and the initial time stamp and ii) stored logic configured to ensure the elapsed time meets or exceeds the development time for the one or more test strips. The one or more sources of electromagnetic radiation can be configured to excite an up-converting phosphor present in the one or more target capture zones of the one or more test strips. The detector can be configured to detect up-converting phosphor emissions from the one or more target capture zones.

In such embodiments, the memory device of the test cartridge is a radio frequency identification ("RFID") tag, the reader/writer of the analyzer is an RFID tag reader/writer, and the RFID tag is configured to include the first batch of information that conveys the information to the analyzer needed to conduct the analysis on the biological sample.

Also provided herein is an analyzing system including, in some embodiments, a sample collector, a test cartridge, and an analyzer. The test cartridge can include a port configured to accept a sample of the sample collector, a microfluidic device, one or more test strips, and a memory device. The microfluidic device can include a filter configured to provide a buffer-diluted plasma filtrate from a feed of a buffer-diluted blood sample. Each test strip can include one or more target capture zones configured to bind one or more targets in the buffer-diluted plasma filtrate. The memory device can include a first batch of information selected from a group consisting of i) information for identifying one or more assays of the one or more test strips, ii) information for determining a development time for the one or more test strips, iii) information for determining concentration of the one or more targets of the buffer-diluted plasma filtrate, iv) information for interpreting assay results for the one or more targets, and v) any combination thereof. The analyzer can include an opening configured to accept the test cartridge, a reader/writer, a processor, one or more sources of electromagnetic radiation, and a detector. The reader/writer can be configured to i) read the first batch of information from the memory device, ii) write a second batch of information to the memory device including an initial time stamp for the buffer-diluted plasma filtrate, and iii) read back the initial time stamp for the buffer-diluted plasma filtrate. The processor can be configured to invoke i) a stored algorithm configured to calculate an elapsed time from an instant time and the initial time stamp and ii) stored logic configured to ensure the elapsed time meets or exceeds the development time for the one or more test strips. The one or more sources of electromagnetic radiation can be configured to excite an up-converting phosphor present in the one or more target capture zones of the one or more test strips. The detector can be configured to detect up-converting phosphor emissions from the one or more target capture zones.

In such embodiments, the memory device of the test cartridge can be an RFID tag, the reader/writer of the analyzer can be an RFID tag reader/writer, and the RFID tag can be configured to include the first batch of information.

Also provided herein is a microfluidic blood-processing device including, in some embodiments, a mixing-reservoir layer, a filter layer, a mesh layer, a channeled layer, and a splitting layer. The mixing-reservoir layer can include a mixing reservoir configured to mix a blood sample and a buffer solution to form a blood-and-buffer mixture. The filter layer can include a filter configured to produce a filtrate of plasma and buffer from a feed of the blood-and-buffer mixture. The mesh layer can include a mesh configured to distribute a plasma-and-buffer filtrate over a substantial area of the mesh. The channeled layer can include a number of channels configured to channel the plasma-and-buffer filtrate to a plasma-and-buffer loading well. The splitting layer can include a number of holes configured to receive and split the plasma-and-buffer filtrate into a number of samples corresponding to the number of holes.

These and other features of the concepts provided herein can be better understood with reference to the drawings, description, and appended claims.

DRAWINGS

FIG. 1 provides a schematic illustrating a biological sample-analyzing system in accordance with some embodiments.

FIG. 2 provides a schematic illustrating an analyzer in accordance with some embodiments.

FIG. 3 provides a schematic illustrating an architecture of the analyzer in accordance with some embodiments.

FIG. 4A provides a schematic illustrating an illumination pathway of an optical sub-system of the analyzer in accordance with some embodiments.

FIG. 4B provides a schematic illustrating a detection pathway of an optical sub-system of the analyzer in accordance with some embodiments.

FIG. 5 provides a schematic illustrating a sample collector and a cartridge in accordance with some embodiments.

FIG. 6 provides a schematic illustrating an exploded view of a cartridge in accordance with some embodiments.

FIG. 7A provides a schematic illustrating a top view of a mixer/splitter/separator ("MSS") in accordance with some embodiments.

FIG. 7B provides a schematic illustrating a layer stack of a MSS in accordance with some embodiments.

FIG. 8 provides a schematic illustrating a lateral flow assay test strip in accordance with some embodiments.

FIG. 9 provides a schematic illustrating a method of a biological sample-analyzing system in accordance with some embodiments.

FIG. 10 provides a schematic illustrating a concentration algorithm in accordance with some embodiments.

FIG. 11 provides a schematic illustrating in accordance with some embodiments.

FIG. 12 provides a schematic illustrating a classification algorithm in accordance with some embodiments.

DESCRIPTION

Before some particular embodiments are provided in greater detail, it should be understood that the particular embodiments provided herein do not limit the scope of the concepts provided herein. It should also be understood that a particular embodiment provided herein can have features that can be readily separated from the particular embodiment and optionally combined with or substituted for features of any of a number of other embodiments provided herein.

Regarding terminology used herein, it should also be understood the terminology is for the purpose of describing some particular embodiments, and the terminology does not limit the scope of the concepts provided herein. Unless indicated otherwise, ordinal numbers (e.g., first, second, third, etc.) are used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. It should also be understood that, unless indicated otherwise, any labels such as "left," "right," "front," "back," "top," "bottom," "forward," "reverse," "clockwise," "counter clockwise," "up," "down," or other similar terms such as "upper," "lower," "aft," "fore," "vertical," "horizontal," "proximal," "distal," and the like are used for convenience and are not intended to imply, for example, any particular fixed location, orientation, or direction. Instead, such labels are used to reflect, for example, relative location, orientation, or directions. It should also be understood that the singular forms of "a," "an," and "the" include plural references unless the context clearly dictates otherwise.

"Biological sample," as used herein, includes any sample ranging from those having classical biological structures to those having structures with one or more biological characteristics such as viruses, satellites, and viroids, as well as proteins such as prions. Biological samples such as the foregoing can be analyzed with the biological sample-analyzing system described further herein.

Unless defined otherwise, all technical and scientific terms used herein have the same meaning as commonly understood by those of ordinary skill in the art.

System

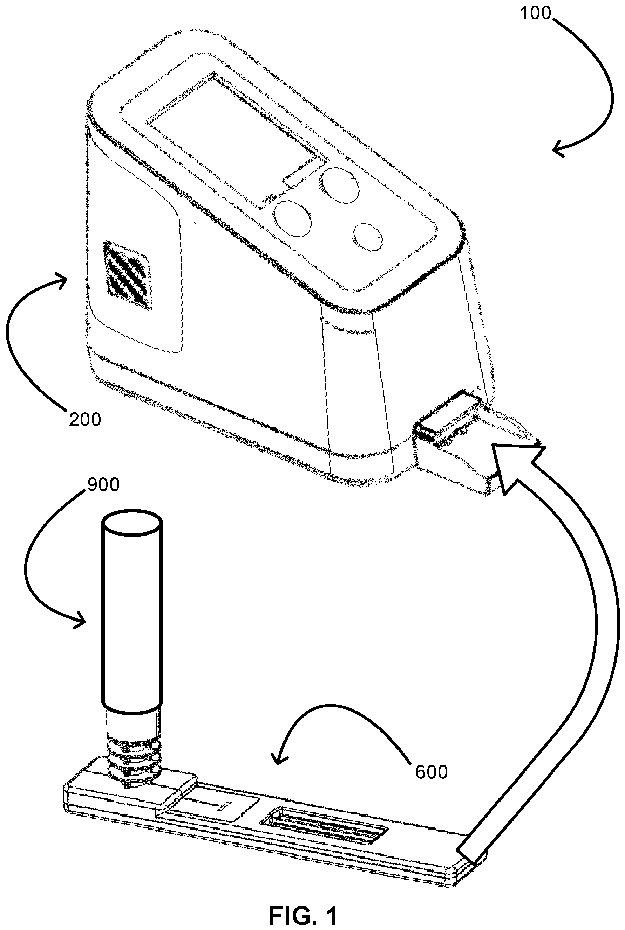

FIG. 1 provides a schematic illustrating a biological sample-analyzing system 100 in accordance with some embodiments.

As shown, the biological sample-analyzing system 100 includes an analyzer 200, a cartridge 600, and a collector 900. Such a system can be a point-of-care analysis system with up-converting phosphor-reporter technology as described further herein.

The biological sample-analyzing system 100 can be configured to measure proteins including prions, as well as other entities such as, but not limited to, spores, bacteria, viruses, satellites, and viroids, which is valuable in many situations. Examples of such situations include during an unexpected exposure to radiation (e.g., ionizing radiation) or some other harmful chemical or biological agent, or during a spread of an epidemic. Other examples include environments that are removed from a hospital or a clinic such as a pharmacy, a home, a remote village, or a town. Yet other examples include the area of veterinary medicine, as well as the areas of botanical and agricultural sampling. Need for such a system exists in many industrial operations such as, but not limited to, chemical plants, water management centers, and sewage treatment centers. An example of this need at the water management center is the need to check for spores in the water. If spores are found, the water management center can alert its users and quickly correct the situation. An example of the need at the sewage management center is the need to check for the presence of certain types of molecules that are found in drugs that are consumed by humans. If left untreated, these molecules may be consumed by water-based wildlife endangering the health of not just the wildlife but also of humans who consume the wildlife. The sewage management center may decide to alter its treatment processes based on the results of such tests. Thus, there are numerous situations in need of such a system that analyzes biological samples.

The biological sample-analyzing system 100 system can include the compact, optics based analyzer 200, the cartridge 600 configured to be inserted into the analyzer 200, and the collector 900 configured for collecting the biological sample. The cartridge 600 includes one or multiple strips with each strip containing locations (e.g., target capture zones such as stripes) where probe molecules are deposited. These probe molecules are designed to have affinity to specific target molecules or analytes. Each probe molecule may also be coupled to an up-converting phosphor particle. When light at a certain frequency is incident upon these up-converting phosphor particles, due to the property of the phosphor particles, light at a shorter wavelength is emitted. The intensity of the detected light may be measured and used to calculate the concentration of target molecules or analytes.

The test cartridge 600 can be a single-use cartridge in that the test cartridge is designed to be disposed after just a single use. Each test strip of the one or more test strips can further include a control target capture zone (e.g., stripe). The processor can be further configured to invoke a stored normalization algorithm configured to divide an up-converting phosphor emission from the control target capture zone from the up-converting phosphor emissions from the one or more target capture zones for each test strip. As such, the normalization algorithm can remove effects from manufacturing variances in the test strips, manufacturing variances in the one or more sources of electromagnetic radiation, different levels of battery power, and variances in biological sample volumes.

A processor can be further configured to invoke a stored classification algorithm configured to convert the up-converting phosphor emissions from the one or more target capture zones to a determination of whether a test result is i) positive as a condition under the test is present, ii) negative as a condition under the test is absent, or iii) indeterminable. The processor can be further configured to invoke a stored classification algorithm configured to convert the up-converting phosphor emissions from the one or more target capture zones to a determination of whether a target of the one or more targets is i) present as exceeding a predetermined threshold, ii) absent as falling below a predetermined threshold, or iii) indeterminable. For example, if an individual has been exposed to a dose of radiation, the stored classification algorithm can provide a determination of whether the dose of radiation is greater than a predetermined exposure threshold, less than a predetermined exposure threshold, or cannot be determined as either too close to call or per an assay failure.

In an embodiment, multiple analyzers like the analyzer 200 contain mechanisms that allow these analyzers to be synchronized to the same time. This allows the analyzers to keep track of the assay development time along with providing other benefits. In another concept, the cartridge 600 includes a storage mechanism with read/write capability such as, but not limited to, an RFID tag or chip. The RFID tag includes information that may be read by the analyzer 200. This information may be used in several ways including in the calculation of the concentration of the target molecules and interpretation (e.g., classification) of the test results. In another embodiment, the strips include features that allow for minimization or removal of analyzer-to-analyzer and other factors that may cause errors in measurements.

There are several benefits of the above approach. In one benefit, the concepts provide a point-of-care diagnostic system to detect and quantify the presence of various molecules and entities such as, but not limited to, protein molecules, nucleic acids, viruses, and bacteria in samples of blood, saliva, urine, or other bodily fluids. The analyzer 600 and the associated cartridge 600 have multiple features that provide accurate and reliable results. As an example, features are provided to minimize the impact of analyzer-to-analyzer variation. The concepts described herein may be applied to various situations including, but not limited to, radiation biodosimetry, bio-warfare agent diagnostics, infectious disease diagnostics, and treatment monitoring.

There are a number of lateral flow assays available in the marketplace, but these assays are either limited in their functionality or quite complex and meant for use in a laboratory. A home pregnancy kit is an example of a lateral flow assay that is limited in its functionality in that it provides a simple yes-or-no type of result. In addition, although there are other point-of-care assays and instruments on the market, few, if any, are designed for non-clinical settings. In contrast, the concepts described herein can support a quantitative output. Furthermore, the systems can be located in various settings including clinical and non-clinical settings.

Analyzer

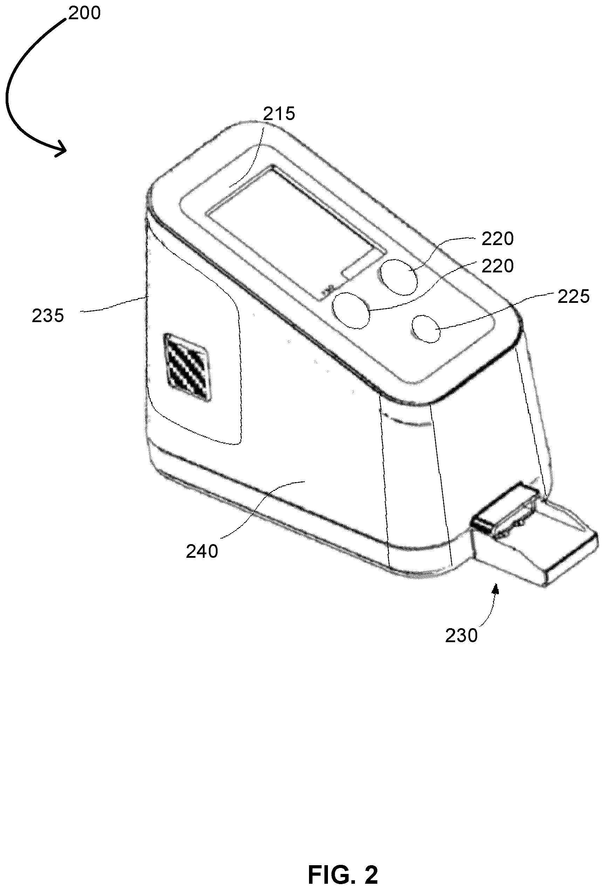

FIG. 2 illustrates a hand-held, point-of-care, up-converting phosphor-reporter technology based analyzer 200. The analyzer 200 includes a display 215, buttons 220, an LED display 225, a cartridge reader 230 or an opening (e.g., slot) with an ambient light-shielding lip to accept the cartridge for reading, a battery compartment 235, a housing 240 that can house various sub-systems including an electrical sub-system and an optical sub-system. Other features and interfaces may be included on the exterior of the analyzer 200 such as, but not limited to, USB interfaces, a power input, a Secure Digital ("SD") card reader; however, for expediency, these are not shown in FIG. 2.

The analyzer 200 may further include a communication interface such as a wireless interface. The processor can be further configured to invoke a communication module such as a wireless module configured to communicate the first batch of information, the second batch of information, and any additional information related to the test cartridge through the communication interface with any of a number of other similar analyzers.

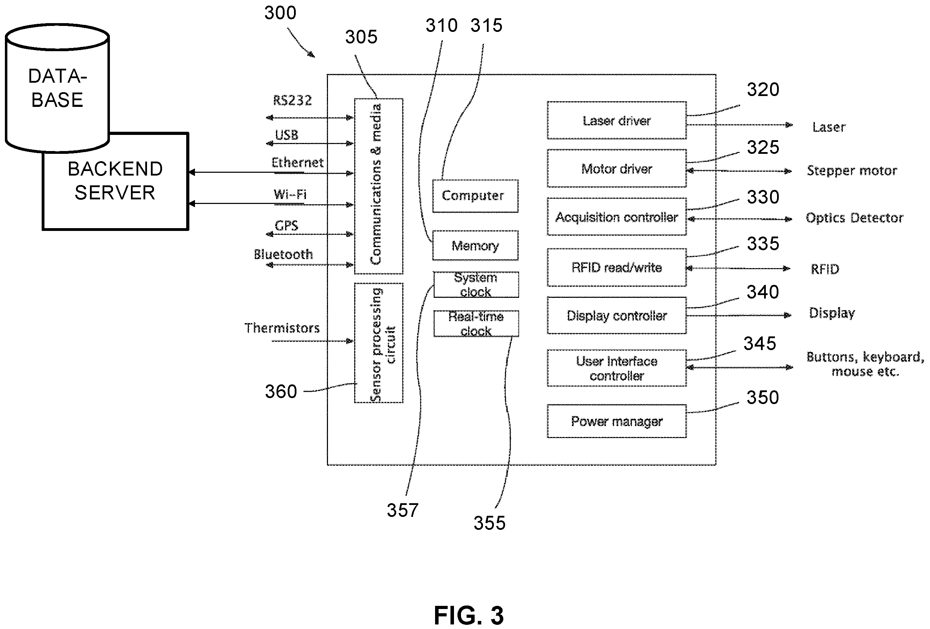

In addition, as shown in FIG. 3, the analyzer 200 is further configured to communicate the foregoing information (e.g., the first batch of information, the second batch of information, and any additional information related to the test cartridge) to a backend database associated with a central server configured to aggregate and organize the information from many other analyzers, as well as sources of related or supporting information (e.g., population data such as demographics, etc.). The analyzer 200, as well as the other analyzers, can subsequently pull such information from the backend database of the central server. Pulling such information can provide important epidemiological data such as: "How many people in this area tested positive to this test?"

In an embodiment, the slot of the analyzer 200 can include a lip configured to shield the detector or measurements thereof from ambient light. The analyzing system can further include a sample collector sized and configured to collect a predetermined volume of the biological sample, the biological sample selected from blood, saliva, and urine.

FIG. 3 illustrates an overall system architecture 300 of the analyzer 200. As shown, the analyzer 200 can include a computer 315, a memory module 310, a real-time clock 355, a system clock 357, a communications module 305 (e.g., a wireless interface operable by means of a wireless module), a laser drive 320, a motor driver 325, an acquisition controller 330, an RFID reader/writer system 335, a display controller 340, a user interface controller 345, a power manager 350, and a sensor processing circuit 360. Any suitable type of computer such as, but not limited to, a central processing unit ("CPU"), a graphics processor unit ("GPU"), and a field programmable gate array ("FPGA") may be used. The memory module 310 can be one of various types including volatile and non-volatile memory. The system clock 357 coordinates the activities of the various sub-systems. The real-time clock 355 receives information from the geo-positioning system ("GPS") receiver that is included in the analyzer 200, so that a common time such as the coordinated universal time ("UTC") is available to all the analyzers in operation at a site. Concepts related to the real time clock and synchronization will be described later. The communication module 305 can communicate in several ways including through RS232, USB, Ethernet, Wi-Fi, Bluetooth, or two or more of the foregoing. Although the communication module 305 is shown as one block, it may also be distributed across several modules to communicate information between analyzers such as the first batch of information described herein, the second batch of information described herein, or any additional information related to the test cartridge through the communication interface. A processor may be configured to invoke a communication module configured to establish a communication channel between the analyzer and one or more additional analyzers to communicate the first batch of information, the second batch of information, or both batches of information related to the test cartridge through the communication interface and over the communication channel with the one or more additional analyzers. The laser driver 320 provides the electronics to drive the lasers. The acquisition controller 330 provides the electronics to detect the emissions from the phosphor particles. The motor driver 325 is responsible to control the position of the cartridge. The RFID reader/writer controller 335 provides the read and write capability to the RFID tag on the cartridge 600. Additional concepts related to the RFID read/write capability will be described later. In alternative embodiments, the read/write capability can be done via near-field communication ("NFC") with a combination of NFC identification tags and reader/writer devices for reading and writing to the NFC identification tags. The read/write capability can also be done via bar codes with a bar code reader/writer device in the analyzer or connected to the analyzer for writing and reading bar codes to the test cartridges. The display controller 340 controls the content of the display 215. The user interface controller 345 reads the inputs from the buttons and other devices. The power manager 350 distributes the power to the necessary sub-systems.

The reader/writer (e.g., the RFID reader/writer 335) of the analyzer 200 can be configured to i) read a first batch of information from a memory device of the cartridge 600, which first batch of information is detailed further herein; ii) write a second batch of information to the memory device of the cartridge including an initial time stamp for a development time of the biological sample on a lateral flow strip, which development time is also detailed herein; and iii) read back the initial time stamp for the biological sample.

A processor of the computer 315 can be configured to i) invoke a stored algorithm configured to calculate an elapsed time from an instant time and the foregoing initial time stamp and ii) invoke stored logic configured to ensure the elapsed time meets or exceeds the development time for one or more test strips in a cartridge.

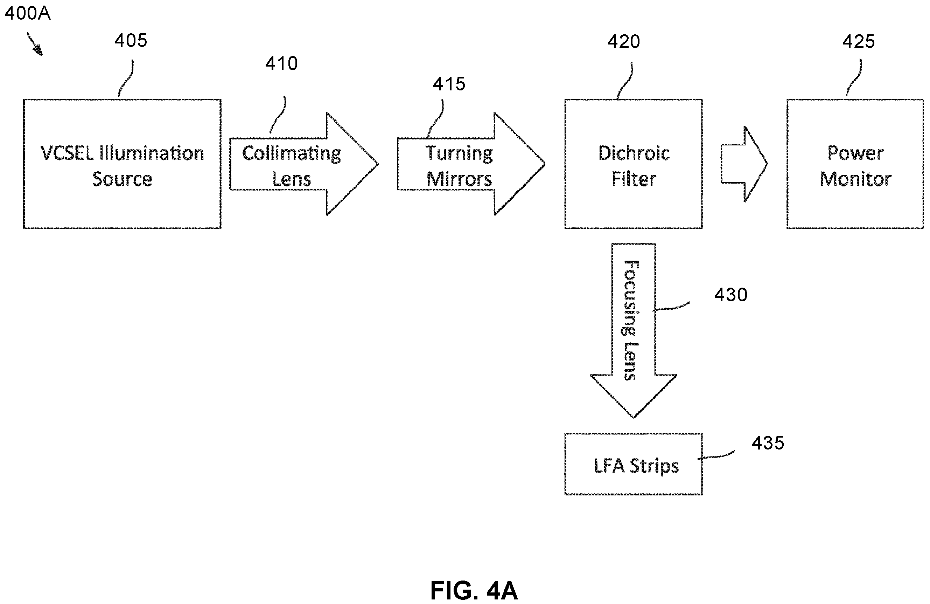

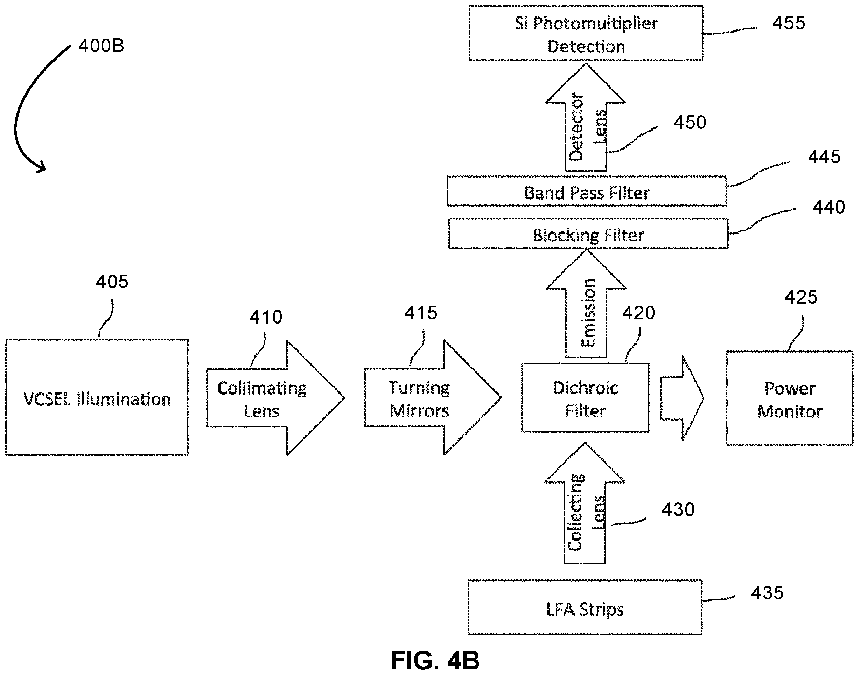

The optical sub-system is illustrated in FIGS. 4A and 4B. FIG. 4A illustrates the block diagram for an illumination pathway 400A whereas FIG. 4B illustrates the block diagram for a detection pathway 400B. In these figures, box 405 illustrates one or more sources of electromagnetic radiation as the laser illumination source. In one configuration of the analyzer 200, a vertical-cavity surface-emitting laser ("VCSEL") emitting light at or around 980 nm, is utilized. Several advantages are realized by using a VCSEL array such as higher reliability, lower cost, lower power consumption, and the fact that an array of sources can be utilized to produce line illumination. The light from the array of sources is then collimated with a collimating lens 410 followed by the passage of the collimated light through turning mirrors 415. The function of the turning mirrors is to achieve alignment of the collimated laser light to the axis of the various optical elements. The light then passes through a dichroic filter 420. The dichroic filter 420 is designed such that some portion of the energy such as, but not limited to, ≤ 1%, is allowed to pass through to a power monitor 425. Most of the energy from the VCSEL illumination source is diverted through a focusing lens 430 to be incident on the assay test strips. The focusing lens 430 focuses the light from the laser such that the strip is uniformly illuminated in a line. Depending on the presence of target molecules, the probe molecules with the up-converting phosphor particles, will bind to the target molecules. Upon illumination from the laser source, these up-converting phosphor particles or up-converting phosphor are excited and emit light at a shorter wavelength. FIG. 4B illustrates the path of the light emitted by the phosphor particles. This light passes through the collimating lens 430 and through the dichroic filter 420. Next, blocking filter 440 filters out light generated by the laser source that is reflected by the dichroic mirror into the detection path. The light from the blocking filter then passes through a band-pass filter 445 and through the detector lens 450 to the silicon photomultiplier detection ("SiPM") array where the light or emissions is detected.

Note, multiple illumination sources may be utilized. As detailed below, the cartridge 600 may have one or multiple assay test strips. Each strip may be illuminated by its own illumination source. Thus, the laser drive 320 illustrated in FIG. 3 may drive one or multiple sources. One advantage of having multiple sources is that only the necessary source needs to be turned on while the others are turned off. This reduces the instantaneous power needed from the battery in addition to reducing the background clutter information that may arise due to cross-illumination. In other words, the possibility of receiving a signal from a strip that was not intended to be scanned or read, is reduced. Another advantage is that having multiple sources provides the flexibility to choose a different wavelength of light if required.

Cartridge

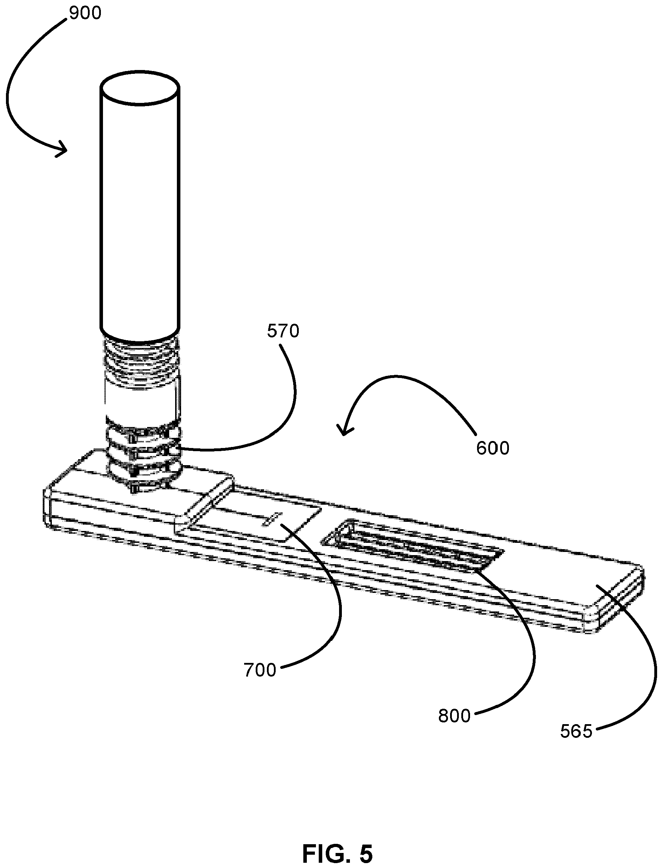

FIG. 5 illustrates the sample collector 900 and the disposable, single-use cartridge 600 that may be used in conjunction with the analyzer 200 of FIGS. 2 and 3. Although the figure does not illustrate the following, multiple types of cartridges may be designed and used with the analyzer 200. One type of cartridge called the "test cartridge" may be used to collect a sample such as, but not limited to, a blood sample from a human or non-human subject, process the blood sample, and provide mechanisms to determine the presence of target molecules in the blood sample. Samples may also be collected from various other life forms or environments such as, but not limited to, plants, water supply systems, and industrial centers. The mechanisms to determine the presence of target molecules will be described in detail below. Another type of cartridge called the "system check cartridge" may be used to check if the analyzer 200 is working within its expected operating range. The system-check cartridge can include one or more test strips with a plurality of standard target capture zones with up-converting phosphor ranging from a low-end standard to a high-end standard of the analyzing system's dynamic range. Additional information on this type of cartridge will also be explained in a later section. Both types of cartridges share a number of features. For clarity, where a distinction needs to be made between the two types of cartridges, the terms "test cartridge" or "system check cartridge" are used. Where no distinction needs to be made, no special term is used. In alternative embodiments, the test cartridge can be configured to analyze the analyzer's dynamic range as well by incorporating features of the system check cartridge.

The analyzing system can be configured to ensure up-converting phosphor emissions are present at the expected levels for the standard target capture zones and are repeatable within an acceptable level of variance. Each test strip of the one or more test strips can further include a control target capture zone. The one or more target capture zones are configured to bind one or more targets of the biological sample. The processor can be further configured to invoke a stored normalization algorithm configured to divide an up-converting phosphor emission from the control target capture zone from the up-converting phosphor emissions from the one or more target capture zones for each test strip. As such, the normalization algorithm can remove effects from manufacturing variances in the test strips, manufacturing variances in the one or more sources of electromagnetic radiation, and different levels of battery power.

Also, the slot of the analyzer 200 can include a lip configured to shield the detector or measurements thereof from ambient light.

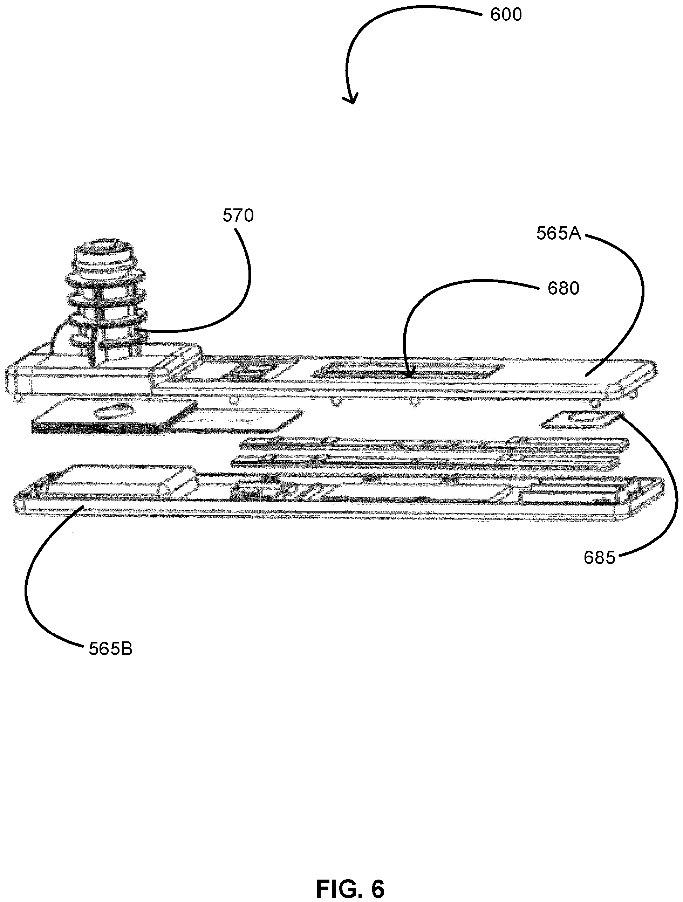

FIG. 5 shows the sample collector 900 and the cartridge 600 including a mixer/splitter/separator ("MSS") 700, two assay test strips 800, cartridge housing 565, and an inlet port 570 configured for deposition of sample from the sample collector 900. FIG. 6 provides an exploded view of the some of the components of the cartridge 600. The structure of the cartridge is supported by a top housing 565A and a bottom housing 565B. The top housing includes the inlet port 570 and two slit openings 680 through which optimal measurements are performed. The MSS is placed below the inlet port and between the top and the bottom housing. Other components that are located between the top and the bottom housing include the two assay strips 800 and a memory device including, but not limited to, one or more RFID tags such as RFID tag 685.

The one or more RFID tags can be selected from read only RFID tags; read-write RFID tags; write once, read many ("WORM") RFID tags; passive RFID tags of the foregoing, and the like. The inclusion of the RFID tag 685 provides multiple benefits. In one benefit, each cartridge can be uniquely identified. Thus, a cartridge can be uniquely associated to a patient or a subject. In another benefit, the RFID tag can provide a way to keep track of the time for the assay to develop completely. The RFID tag can also contain information that the analyzer can use to perform computations such as, but not limited to, identifying the assay type, finding the concentration of the target molecules, and interpreting the result (e.g., a positive or negative test). Thus, the memory device or RFID tag can be configured to include a first batch of information selected from a group consisting of i) information for identifying one or more assays of the one or more test strips, ii) information for determining a development time for the one or more test strips, iii) information for determining concentration of the one or more targets of the biological sample, iv) information for interpreting assay results for the one or more targets, and v) any combination thereof. In an embodiment, the first batch of information includes all four of i) the information for identifying the one or more assays of the one or more test strips, ii) the information for determining the development time for the one or more test strips, iii) the information for determining the concentration of the one or more targets of the biological sample, and iv) the information for interpreting assay results for the one or more targets, so that the analyzer does not need to have prior knowledge of the biological sample being tested.

A battery source may be included in the cartridge 600. This battery source may be used for various purposes such as, but not limited to, powering an LED light that may, in turn, be used to convey a warning, alert, or a message.

In one additional detail, the cartridge can include teeth along one or both longitudinal edges of the cartridge as part of the top housing 565A, bottom housing 565B, or both. These teeth engage with a gear wheel within the analyzer that may be driven by a stepper motor. In operation, a laboratory technician may insert the cartridge into the analyzer. The stepper motor steps the cartridge in an outward direction while the laser illuminates the strips. This is followed by the detection of the up-converted signal by the detector when the cartridge is not in motion.

MSS

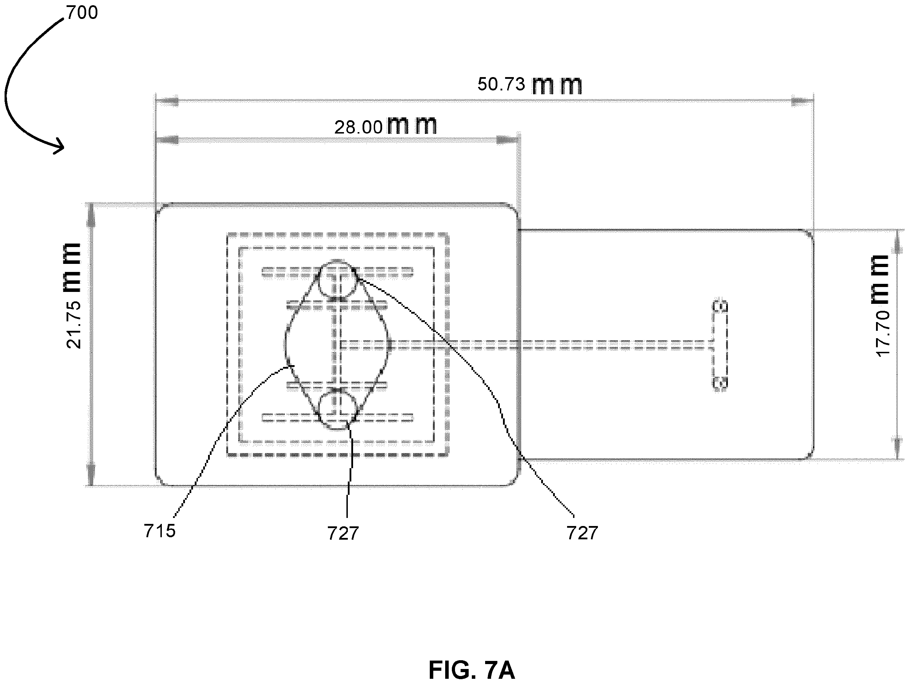

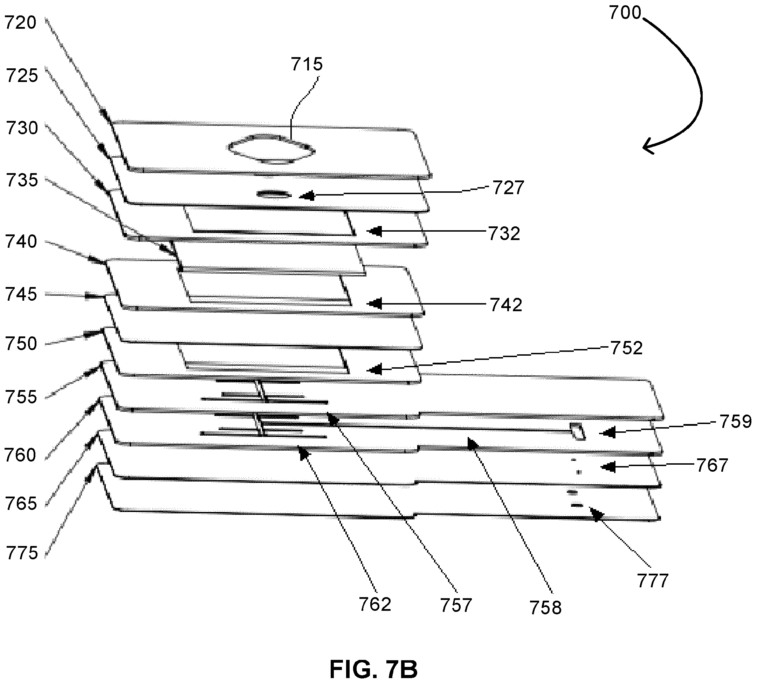

FIGS. 7A and 7B provide the MSS 700 that takes blood and buffer as its input, mixes them, separates the plasma from the blood, and splits the plasma and buffer into two separate pools. FIG. 7A describes a top view of the MSS 700. Representative dimensions are shown in the FIG. 7A, but other dimensions are also possible. FIG. 7B shows an exploded view of the MSS 700.

The MSS 700 is a microfluidic device composed of a number of layers; the functionality of these layers will now be further described. Blood deposited by the collector 900 enters the MSS 700 and passes through a cutout 715 in layer 720. Layer 720 can be made of a material such as, but not limited to, polyethylene terephthalate ("PET"). The blood drops through the cutout 715 and falls on top of layer 725, which layer 725 can also be made from PET. Following the expulsion of blood, a buffer held in the reservoir 715 is also expelled and drops through cutout 715 onto layer 725, where it mixes with the blood to create a blood-and-buffer mixture. This blood-and-buffer mixture then passes through the holes 727 in layer 725 and through the layer 730 that acts as a spacer to then reach the filter 735. The layer 730 acts as a spacer by having a cutout 732 in the material of the layer (e.g., PET). The filter 735 can be an asymmetric filter that has different pore sizes in the input and output of the filter, wherein the larger pores are placed at the input and the smaller pores provide the output. This filter 735 is designed to trap red blood cells or erythrocytes, white blood cells or leukocytes, and platelets of the blood in the blood-and-buffer mixture but allow the plasma of the blood and the buffer to flow through it. Such a filter includes, but is not limited to, a Vivid™ Plasma Separation Membrane from Pall Corporation, Port Washington, New York. Plasma and buffer of a plasma-and-buffer mixture will then pass through the spacer layer 740. This spacer layer 740 is similarly formed as described above and the cutout is labeled as 742. The plasma-and-buffer mixture now encounters mesh layer 745. The purpose of the mesh layer 745 is to distribute (via wicking, pressure generated from the plunger, and/or combination of both) the fluid by allowing lateral motion due to its woven structure. Using a mesh such as this, allows the full footprint of the filter 735 to be utilized for maximizing its ability to filter blood with a small thickness profile. The mesh is a very thin material of a thickness of about 0.003" thickness, which reduces the amount of volume lost compared to conventional method of providing support to filter membranes. The mesh can be made of various materials such as but not limited to nylon, polyester, polypropylene, Stainless Steel, and/or Steel. The mesh layer separates the fluids evenly so that the fluids eventually go into each channel equally. Once the plasma-and-buffer mixture passes through mesh layer 745, it then passes through the spacer layer 750 that is made similarly as described above. The cutout in this layer is labeled as 752. The plasma-and-buffer mixture now flows through layer 755, which is also a spacer layer however the shape of the cutouts 757 in this layer match the shape of the channels in layer 760. Layer 755 can be considered an optional layer in some embodiments as one of its function is to prevent the mixture coming in direct contact with adhesive that can be applied to layer 760. However, in some other embodiments, layer 755 can be considered a requisite layer as it provides the foregoing function as well as a ceiling for one or more of the channels thereunder. The filtered plasma and buffer then drop into channel 762 formed within layer 760. This channel is designed to maximize the use of the entire footprint of the mesh filter by extending to the edges of the footprint. Once the plasma and the buffer are driven into channel 762, it flows through the stem 758 of the channel into a cutout 759 where it falls onto layer 765. The mesh layer 745 permits lateral fluid flow while minimizing the dead volume of the plasma-and-buffer filtrate. The mesh layer 745, cutouts 757, and channel 762 work to accomplish the same purpose.

Note, however, it is possible to accomplish that function with just the features from cutouts 757 and channel 762 without the mesh layer 745 but this will cause more dead volume because more channels would be needed maximize the usable footprint of the filter. The plasma and buffer are then sent through two holes, labeled collectively as 767, on layer 765. These holes are precision cut and can be specified to have diameters that are within +/- 5% of each other. The precision of these holes enables the splitting of the plasma and buffer solution into two volumes that can be within 10% of each other. Layer 775 is an adhesive layer that is meant to adhere to the housing of the device 700. The holes 777 can be larger than holes 767 to ensure that the adhesive from layer 775 does not interfere with the holes 767. The holes 767 are sized to be small (about 0.25 mm) so that they are the primary resistances in the path of the fluid. The smallness of the holes minimizes the contributions of variations in channel shape and size. The split samples now exit the holes 777 and fall onto a suitable collection area.

Adhesive such but as, but not limited to, pressure sensitive adhesive, can be used to keep the various layers together and to build the structure of the filter. Thus adhesive can be applied to the top and bottom of layer 720. The adhesive on top of the layer 720 can be used to adhere to the housing of the cartridge 600. No adhesive is applied on layer 725. Adhesive is applied on both sides of layer 730, 740, 750, and 760. No glue is applied to the filter 735 or the mesh filter 745.

Thus, with this configuration, example volumes of blood, plasma, and buffer are described below. Starting with, for example, 33 $\mu$L of blood and 440 $\mu$L of buffer that fall into the cutout 715, after the filter 735, the plasma to solution ratio can be 1:20. (Note this accounts for the initial volume of material. The volume that is in the buffer chamber of the sample collector. What makes it into the MSS is a number smaller than that since there is some dead volume in the path from the buffer chamber to the MSS.) After the sample is split, about 75 $\mu$l of solution can be deposited into suitable collection areas for further processing. These are example values and other values are possible.

A variant of the MSS 700 is now described, which variant assembly will enable filtration of whole blood to produce undiluted plasma. In this configuration, only layers 730, 735, 740, 745, 750 are included. The layers 720, 725 responsible for mixing are removed because there is no need to mix whole blood with buffer in this configuration. The layers 755, 760, 765, and 775 responsible for splitting the sample are removed because there is no need to deposit samples into separate sample wells. This assembly is then sandwiched between two rigid pieces of plastic (e.g., the top housing 565A and the bottom housing 565B of the cartridge 600) with holes in each to allow the sample to enter into and exit the assembly. These rigid pieces of plastic allow sufficient structural support so that when the assembly is pressurized it does not "balloon" and cause an unwanted distribution of blood. In this rigidly supported configuration, the spacing between the rigid plastic on the sample input side and the filter 735 does not change significantly with pressure which allows the fluid to reach the edges of the filter instead of pooling in the center when there is no rigid support (i.e., the assembly does not turn into a bowl-like structure when properly supported). A specific amount of whole blood is inserted into the assembly that is related to the total area of the filter membrane so that it is not overloaded with too much blood. The pressure supply is set to a low pressure (-2.5 psi) to drive blood and wet the entire footprint of the filter. In the course of about 1 minute, undiluted plasma exits the filter 735 and then moves laterally through the mesh, and finally out the exit hole of the rigid plastic support to be collected. This variant assembly borrows features from the MSS 700 described above in FIGS. 7A and 7B, but this assembly replaces the mixing and splitting features with rigid supports to promote the full use of the filter's area to keep spacing between layers small and consistent. In this configuration, the total whole blood volume can be lower since it depends on the area of the filter. However, the total volume of blood can be adjusted as desired, for example, to a manufacturer's specifications based on an exposed area of the filter. For Example, the GR-grade Vivid™ Plasma Separation Membrane from Pall Corporation for whole blood recommends 40-50 $\mu$L of whole blood per square centimeter of the membrane.

Thus, the concepts and the configurations above provide techniques to process diluted or whole blood and to provide the functions of mixing and splitting as required.

Test strips

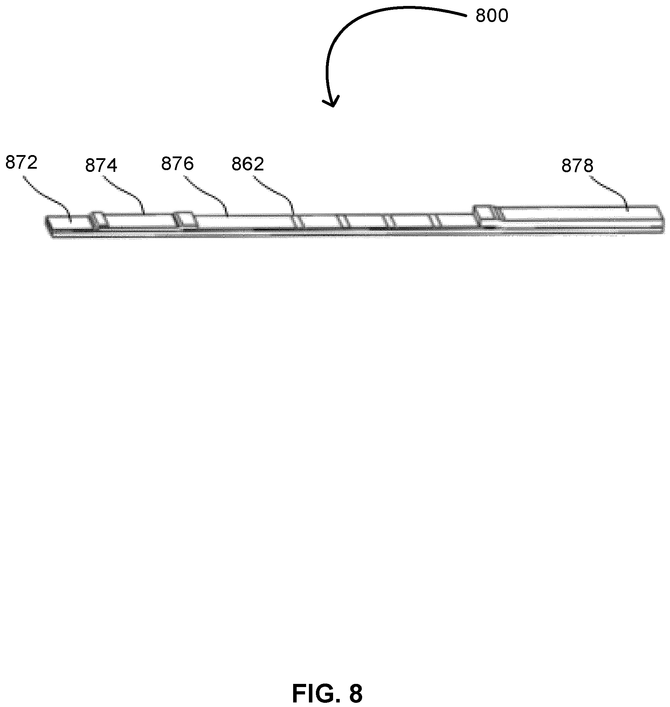

FIG. 8 illustrates a lateral flow assay test strip 800 separately. The test strip 800 may be made of several components including a sample application pad 872, a conjugate pad 874, and a nitrocellulose membrane 876 that includes one or multiple target capture zones such as 862. The sample application pad is included to distribute the sample evenly on top of the conjugate pad. The conjugate pad contains the reporter particles that are used to tag the target molecules or analytes. As the sample flows through the conjugate pad, the reporter particles solubilize and flow with the sample. Several types of reporter particles may be utilized including, but not limited to, phosphor-based reporters. These phosphor reporter particles are coated antibodies called reporter antibodies. The target capture zones are locations where probe molecules may be immobilized. At the far end away from the sample application pad, the test strip may also include an absorption pad so that liquid samples remain confined within the structure of the cartridge 600.

Again, each test strip may contain one or several target capture zones, where each target capture zone contains immobilized probe molecules. In case multiple target capture zones are included in a single test strip, each target capture zone may contain a different probe molecule. Subsequently, each target capture zone can bind to a different target molecule. This concept of capturing different target molecules can be called multiplexing. The concept of multiplexing may be extended to capture different types of target molecules with different types of assays. The cartridge 600 illustrated in FIGS. 1, 5, and 6 has two test strips. Thus, each test strip may be designed to perform a different type of assay. As an example, one test strip may be designed to perform a sandwich assay where the target molecule is "sandwiched" between two antibodies. In this type of assay, the intensity of the up-converted light is proportional to the concentration of the captured target molecules. The other test strip may be designed to perform a competitive assay. This type of assay may be used when the concentration of the target molecule in the sample is inherently high. In this case, instead of immobilizing antibodies on the target capture zones, the target molecules themselves are immobilized at these locations. Similar to the situation with the sandwiched assay, the reporter particles bind to the target molecules prior to reaching a target capture zone; however, if the concentration of the target molecules is higher than an already normally high value, then most or all available sites on the phosphor particle may bind to the target molecules. When this occurs, no binding or very few phosphor particles bind to the immobilized target molecule on the target capture zones. If the concentration of the target molecule is within the normal range but still high, some sites on the phosphor molecule may still be available and bind to the immobilized target molecule on the target capture zone. Thus, the lower the concentration, the higher the up converted signal will be from the target capture zones. Thus, with these and other types of similar configurations, it can now be seen how multiplexing may be achieved. A single cartridge may include one or multiple sites through which the concentration of one or multiple proteins can be determined. In addition, one or multiple types of assays can be used to capture the different target molecules.

FIG. 5 illustrates the cartridge having two lateral flow assay test strips, but, more generally, there may be more or fewer. The choice of the number of test strips may be determined by several factors including the need to separate out the test molecules in two test strips to achieve better signal to noise ratio. Each test strip includes one or multiple test sites or target capture zones that are incorporated or encoded with the probe molecules. As shown in the figures, these test sites or zones may have the shape of a transverse stripe running across the width of the test strip, although other shapes are not excluded. Each transverse stripe may be encoded with a different probe molecule so that different target molecules are captured when the filtered plasma wicks down the test strip.

The lateral flow assay test strips can include a control site or control target capture zone. The control site or zone, which can also be a transverse stripe running across the width of the test strip, may be used to minimize or remove the effect of the various factors that can cause errors in the calculations including analyzer-to-analyzer variances of the analyzers, variances in manufacturing of the test strips, variances in laser brightness, and variances in battery potential. As was stated earlier, the probe molecules located at the test sites capture the target molecules. When laser light of a certain frequency shines at these test sites, the up-converting phosphor captured at these sites emits light at a shorter wavelength. The intensity of the up-converted light is used to calculate the concentration of the captured target molecule. However, a number of factors can cause variance in the intensity; thus, to minimize or remove the effect of these factors a control site is included on the test strip. The control site is immobilized with antibodies that have a strong affinity to the reporter antibodies that are coupled to the UPT particles. Thus, the control site captures the reporter particles whether or not the target molecules are present in the sample such that it provides a measure of the mass (or number) of reporter particles that flowed through the membrane. This value is used to normalize the measured intensities from the other test strips. Additional details about how this normalized intensity is used to calculate target molecule concentration is given below; however, this process of normalization provides the benefit of removing or reducing the effect of the factors that may cause variation of the measurements such as variances in components such as laser output, battery potential, variances in manufacturability of test strips, and the like.

The control site has yet another function. In one configuration, the control site is located at the end farthest from the inlet port 570 and the conjugate release pad. By measuring light emitted from the UPT in the control site, the analyzer confirms that the sample has reached the end of the test strip. If the analyzer determines that the control site emission is below a threshold determined by testing to indicate the reporter particles did not flow over the test strip properly, an error is flagged, and a failure message may be displayed. In addition, information about this error condition may be written into the RFID tag.

System Check Cartridge

It was mentioned earlier that multiple types of cartridges may exist. A system check cartridge may be designed to provide a system check function. In this concept, the system check cartridge may include one or more lateral flow assay strips with multiple standard target capture zones, each target capture zone coupled to a known concentration of the reporter particle. The multiple target capture zones can include up-converting phosphor ranging from a low-end standard to a high-end standard of the analyzer's dynamic range.

Again, the system check cartridge includes substrates with target capture zones of the up-converting phosphor particles at concentrations ranging from the low end to the high end of the analyzer's dynamic range. When the system check cartridge is inserted, the analyzer will repeatedly make optical measurements and ensure that the signals obtained from each target capture zone are present, are at the expected levels, and are repeatable with an acceptable level of variance. It will also ensure that the signals are linear with the concentration of the up-converting phosphor particles. If any of these error checks fail, a warning or fault message may be communicated. As such, the analyzer is configured to ensure up-converting phosphor emissions are present at the expected levels for the standard target capture zones and are repeatable within an acceptable level of variance.

Sample collector

There is need to collect a biological sample such as a sample of blood, saliva, urine, or other bodily fluids. For example, a blood sample can be collected from a technique such as a finger prick, and the blood sample can be deposited in the cartridge 600. The analyzer 200 can subsequently perform multiple assays.

Also provided herein is a blood-processing test cartridge including, in some embodiments, a port configured to accept a sample from a sample collector; a blood-processing assembly including a filter configured to provide a buffer-diluted plasma filtrate from a feed of a buffer-diluted blood sample; and one or more test strips. Each test strip can be configured with one or more target capture zones to respectively bind one or more analytes in the buffer-diluted plasma filtrate.

Also provided herein is a microfluidic blood-processing device including, in some embodiments, a mixing-reservoir layer, a filter layer, a mesh layer, a channeled layer, and a splitting layer. The mixing-reservoir layer can include a mixing reservoir configured to mix a blood sample and a buffer solution to form a blood-and-buffer mixture. The filter layer can include a filter configured to produce a filtrate of plasma and buffer from a feed of the blood-and-buffer mixture. The mesh layer can include a mesh configured to distribute a plasma-and-buffer filtrate over a substantial area of the mesh. The channeled layer can include a number of channels configured to channel the plasma-and-buffer filtrate to a plasma-and-buffer loading well. In an embodiment, the channeling layer has a ceiling layer, a floor layer, and the channel cutout layer. The splitting layer can include a number of holes configured to receive and split the plasma-and-buffer filtrate into a number of samples corresponding to the number of holes. In an embodiment, the splitting layer may be formed from multiple distinctly formed layers but combine to achieve the purpose of the splitting layer.

Also provided herein is a microfluidic blood-processing device including, in some embodiments, a filter layer, a mesh layer, and a housing. The filter layer can include a filter configured to produce a filtrate of plasma from a feed of the blood sample. The mesh layer can include a mesh configured to distribute a plasma filtrate over a substantial area of the mesh. The housing can be configured to house the mesh layer and the filter layer between opposing housing members of the housing, the housing members including openings for loading the blood sample on the mesh and collecting the plasma filtrate from the filter layer. The housing can provide structural integrity to the blood-processing device for application of pressure to the blood-processing device for filtering the blood sample under the pressure.

Also provided herein is a method of a microfluidic blood-processing device including, in some embodiments, mixing a blood sample and a buffer solution to form a blood-and-buffer mixture in a mixing reservoir of a mixing-reservoir layer of a blood-processing device; producing a filtrate of plasma and buffer from a feed of the blood-and-buffer mixture with a filter of a filter layer of the blood-processing device; distributing a plasma-and-buffer filtrate over a substantial area of a mesh of a mesh layer of the blood-processing device; channeling the plasma-and-buffer filtrate to a plasma-and-buffer loading well with a number of channels in a channeled layer of the blood-processing device; and splitting the plasma-and-buffer filtrate with a number of separating holes in a splitting layer of the blood-processing device.

Methods

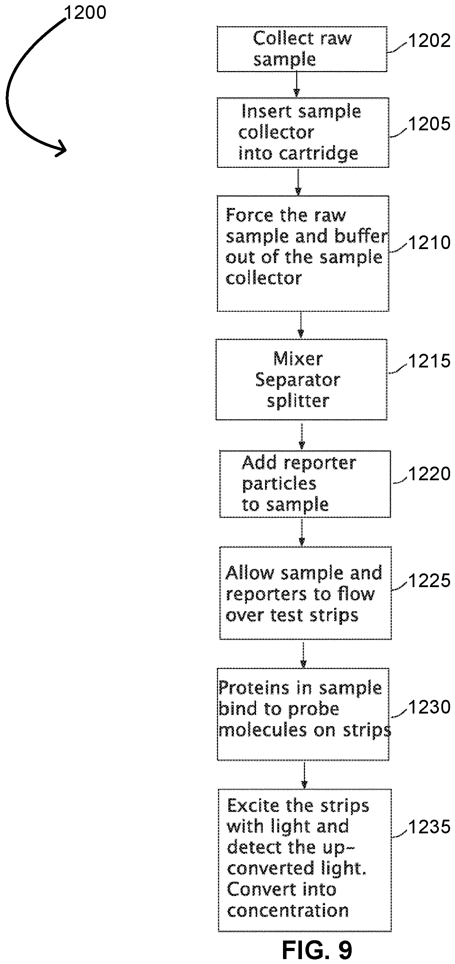

The testing process 1200 is now generally described in FIG. 9. In box 1202, the testing process is initiated by collecting a sample with the sample collector such as the sample collector 900. Specific configurations of the sample collector depend upon the type of sample (e.g., saliva, urine, etc.) and where it is collected from (e.g., human, non-human animal, environment, etc.). For example, a sample of saliva may be obtained with a cotton swab that is subsequently dipped in a buffer solution. The sample collector here may have a mechanism to accept the swab. A sample from a plant may be obtained by crushing plant material and soaking the material in a buffer solution. The sample collector here may have a mechanism to accept the plant material along with a mechanism to crush the material. Each of these sample collectors may have a common feature that enables these collectors to couple to the cartridge. Thus, after the sample is collected, in box 1205, the sample collector may be inserted into the cartridge at the location of the inlet port. FIG. 5 illustrates the sample collector 900 coupled to the cartridge 600. After insertion, in box 1210, the sample or more in particular the raw sample, and an optional buffer are then forced to flow through the MSS 700, that mixes the raw sample with the buffer, filters out the cellular components, and splits the sample into two filtered portions. The process of mixing, separating, and splitting is illustrated by box 1215. The two portions of the filtered sample each are then forced through the sample application pad and the conjugate pad of the assay test strips 800. As mentioned earlier, the sample application pad distributes the sample evenly on to a conjugate pad that contains the reporter particles such as the phosphor reporter particles. This action, illustrated by box 1220, solubilizes the phosphor particles. Then in box 1225, the filtered sample and the phosphor particles with the associated reporter antibodies flow over the test strips 800 by capillary action. As indicated above, each test strip includes one or several target capture zones, and each target capture zone thereof is a location where probe molecules such as, but not limited to, another type of antibodies called the capture antibodies, are immobilized. The capture antibodies capture the target molecules contained in the sample as the sample flows over the target capture zones. This action is illustrated in box 1230. The capture antibodies and the reporter antibodies may be the exact same molecule or they may be different molecules altogether. Then in box 1235, the test strips are illuminated with light at a specific wavelength. The phosphor particles that are illuminated emit light at a shorter wavelength. The intensity of the emitted light is converted into a concentration value, which will be described in detail below.

Calculating concentrations of target molecules

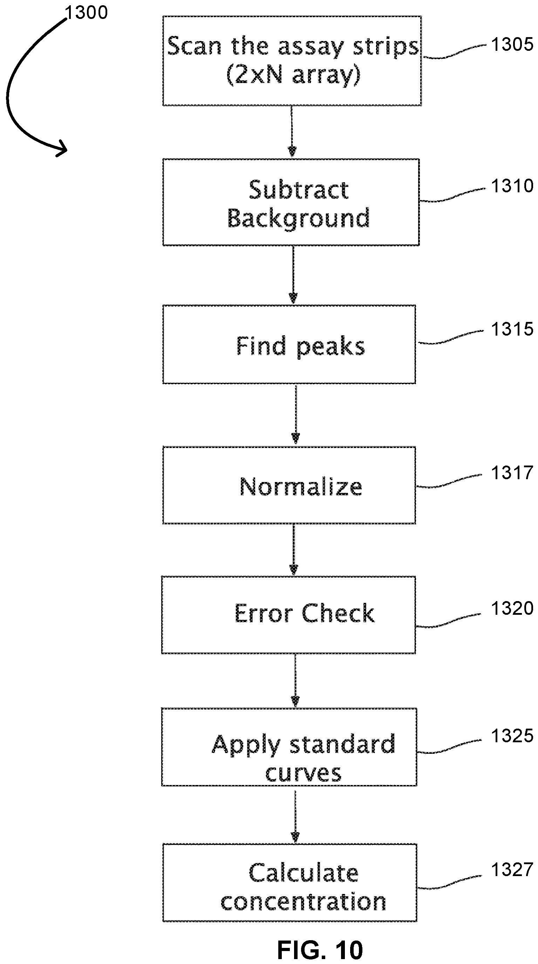

After up-converting phosphor emissions from the one or more test target capture zones are detected, the intensity of the up-converted light is used in an algorithm to calculate the concentration of the target molecules. The detected light is converted to electrical signals and digitized such that the digitized values are proportional to the intensity of the detected light. These digitized values will be referred to by the phrase "intensity readout" herein. The algorithm to convert the intensity readout to concentration will be called the "concentration algorithm" and is illustrated in FIG. 10 as 1300.

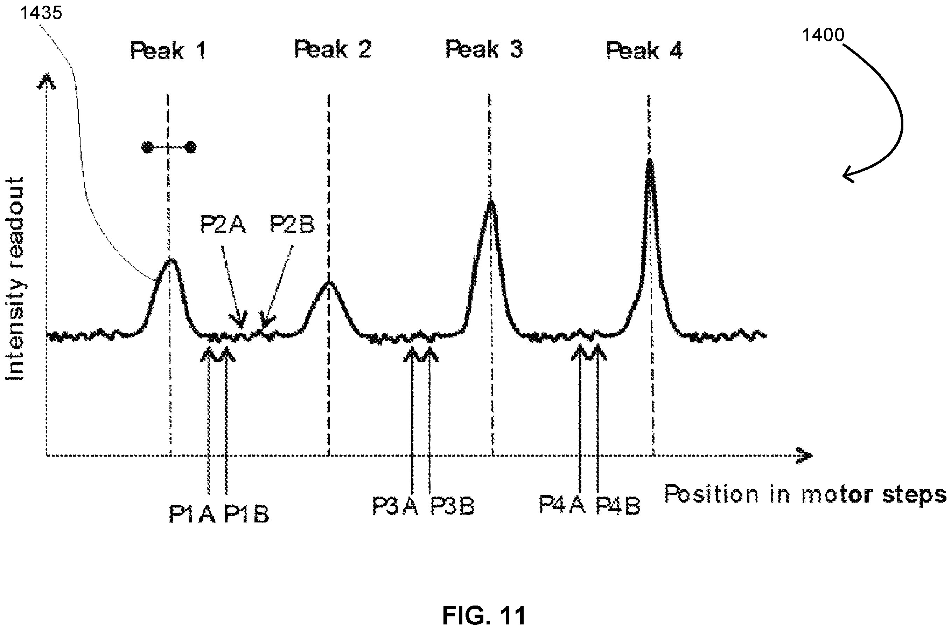

In FIG. 10, box 1305 represents the action of positioning the cartridge, illuminating the test strips with light at an appropriate wavelength, detecting the up-converted light, and obtaining the intensity readout. The "N" represents the number of readout measurements that are obtained for each test strip. Since in the configuration illustrated in FIG. 5, two test strips are shown, the number of readout measurements is 2xN. After completion of the scan, an intensity readout 1400 including a curve such as curve 1435 in FIG. 11 may be obtained. The X-axis in FIG. 11 represents the position of the cartridge 600. The Y-axis represents the intensity readout values. The curve 1435 is illustrated with four peaks labeled Peak 1, Peak 2, Peak 3, and Peak 4. These peaks correspond to increases in intensity readout values of the light emanating from test sites and the control site on a test strip being scanned. In this particular example, the test strip has four test sites: three actual test sites and one control site. Thus Peak 1, Peak 2, and Peak 3 are associated with the actual test sites and Peak 4 is associated with the control site. The light intensity from these sites has increased values due to the fluorescence of the up-converting particles coupled to the probe molecules that bind to the target molecules at these locations.

Next in box 1310, the background value is subtracted. As can be seen in curve 1435, the floor of the curve is positive and non-zero. The magnitude of the peaks should be measured relative to the floor of the curve otherwise the magnitude may be overestimated. Several methods may be used to subtract the background value. In one technique, the background value is calculated as the value of the curve at some predefined location on the curve. More than one value may be used to calculate the background value. In addition, different background values may be calculated for each peak. Thus for Peak 1, the value of the curve at P1 A and P1 B may be used as background values. The values at these two locations may be averaged and the averaged value may be subtracted from the value of the curve at Peak 1. Similarly, the values at P2 A and P2 B, P3 A and P3 B, and P4 A and P4 B may serve as background values for Peak 2, Peak 3, and Peak 4, respectively. Alternatively, the background may be removed by taking the first derivative of the curve.

Next in box 1315, a searching algorithm is employed to search for the peaks in the curve 1435. Tolerances during the manufacture of the cartridges may result in the test sites in each cartridge not being in the exact same location relative to the frame of the cartridge. A peak-searching algorithm may be used to accommodate for this variation in the locations of the peaks that arise from variations in the test locations. There are a number of ways a peak-searching algorithm may be implemented. In one technique, approximate locations of where the peaks should be, are provided to the analyzer 200. The peak-searching algorithm may search around this approximate location until it finds a peak. If no peaks are found because of the lack of target molecules, then as part of the error-checking algorithms briefly outlined below, the test may be considered as invalid or indeterminate. In the case of proteins, the tests are arranged to work within a specified reference range that may include abnormally low values.

In box 1317, a normalization algorithm may be applied. Normalization is an advantageous step as it may minimize or remove the impact of variances in the analyzer 600 and among a group of networked analyzers. Normalization results in a ratio and may be achieved by dividing the background subtracted value of the peaks emanating from the test sites by the background subtracted value of the peak emanating from the control site. The processor of the computer 315 in the analyzer 200 can be configured to invoke a memory-stored normalization algorithm configured to divide an up-converting phosphor emission from the one or more target capture zones by the up-converting phosphor emissions from the control zone for each test strip, thereby removing effects from manufacturing variances in the test strips, manufacturing variances in the one or more sources of electromagnetic radiation, and different levels of battery power, some of which can occur by leaving the analyzers, cartridges, or both on a shelf (e.g., in storage) for a time until ready to use again. As such, analyzers such as the analyzer 200 is configured to modify its internal calculations to accommodate variances in components such as laser output, battery life, variances in manufacturability of test strips, and the like. Note, the lasers may be a source of electromagnetic radiation and have variances in their output due to variants in their manufacture. Similar with the battery, the battery may have different levels of battery power. In addition, a time of use of the cartridge is unpredictable so the stored normalization algorithm compensates for the level of battery power at the time of use.

In box 1320, error checking algorithms may be implemented. In general, various types of error checking may be implemented throughout the operation of the analyzer 200. A separate section below outlines some of the error checks. However, in particular to FIG. 10, error checking within the context of computing the concentration, may include ensuring that the background subtracted normalized ratios are within an expected range of values.

In box 1325, the background subtracted and normalized ratios from box 1317 are used to calculate the concentration of the target molecules. To do this, a function that relates the ratios to concentration value is utilized. This function is called a "standard curve." This curve may be different for each target molecule being analyzed. Information about this curve is stored in the RFID tag within the cartridge 600 at time of manufacture, and the information about the curve is read by the analyzer 600 during the computation of the concentration. Since the curve may be different for each target molecule, information for all the necessary curves is stored within the RFID tag. In addition to differing for each target molecule, the curve for a specific target molecule may differ from cartridge to cartridge or from lot to lot, depending on the manufacturing processes.

In one configuration, the information related to standard curves that is stored in the RFID tag, is the set of coefficients in the equation specified below:

$$C = \alpha * (k_0 + k_1\varphi + k_2\varphi^2 + k_3\varphi^3 + k_4\varphi^4) \quad \text{Eqn. 1}$$

In this equation: C is the concentration, $\alpha$ is the units conversion factor, $k_0$, $k_1$, $k_2$, $k_3$, and $k_4$ are the coefficients that may be stored on the RFID tag, and $\varphi$ is the ratio obtained after normalization.

The calculation of Eqn. 1 is performed in box 427. The coefficients $\alpha$, $k_0$, $k_1$, $k_2$, $k_3$, and $k_4$ are stored within the RFID tag for each target molecule.

In alternate configurations, the standard curves may store coefficients for equations other than specified above. In yet other alternate configurations, a look up table ("LUT") may be used to convert a intensity readout value to a concentration. It may also store an LUT that outputs a value of the concentration given a value in the intensity readout. In yet other configurations, other mathematical operations may be performed on the intensity readout values (e.g., curve 1435) prior to calculating the concentration. Examples of the mathematical operations include low-pass filtering.

Application of the analysis system to determine level of exposure to radiation

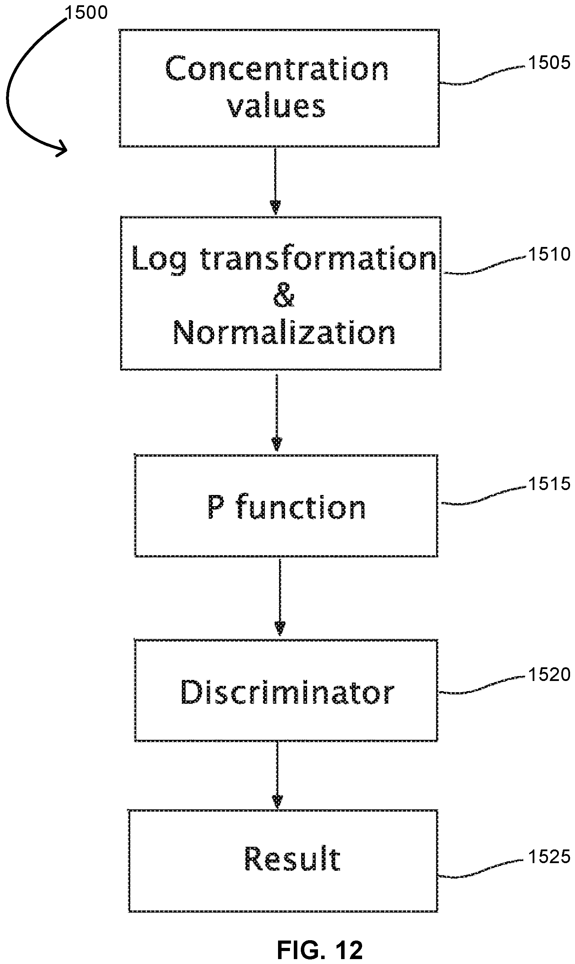

One example of the use of the biological sample-analyzing system 100, is to determine if an individual has been exposed to a radiation (e.g., ionizing radiation) dose at, above, or below a predetermined threshold value. The algorithm that makes this determination will be called a "classification algorithm" to distinguish it from the concentration algorithm described above. The classification algorithm may also be implemented within the analyzer 200 and may be invoked after the concentration algorithm produces the concentration values. The processor of the computer 315 in the analyzer 200 can be configured to invoke a memory-stored classification algorithm configured to convert the up-converting phosphor emissions from the one or more target capture zones to a determination of whether a target of the one or more targets is i) present as exceeding a predetermined threshold, ii) absent as falling below a predetermined threshold, or iii) indeterminable as either too close to call or per an assay failure. The classification algorithm 1500 is briefly explained below and is illustrated in FIG. 12.

In box 1505 of FIG. 12, the concentration values resulting from running the concentration algorithm, is input to the classification algorithm. In box 1510, mathematical transforms may be applied to the concentration values. Various types of mathematical transforms may be utilized; however, log transformation and species normalization (explained below) are found to be advantageous when applied. Log transformation of the concentration values permits the use of standard statistical techniques. Species normalization involves taking the log transformed values of the concentration from a specific animal and dividing it by the average of the log transformed values of the concentration for that specific target molecule, where the average is calculated by measuring the concentration of that specific target molecule from samples obtained from multiple animals at baseline. The baseline case may be defined appropriately as desired and includes, but is not be limited to, animals that are healthy and not exposed to radiation (for this particular example of trying to determine the level of exposure). Species normalization values may also be different for different species of animals. For example, the species normalization value may be different for non-human primates ("NHP") than for humans. Thus, a baseline case for humans may be obtained from concentration of the target molecules in samples from multiple humans known to be unexposed to radiation. The advantage of the species normalization is that it enables cross-species comparison of the concentration values.

In box 1515, the P-function (predictor function), is applied. The P-function may be described as a formula that predicts if a certain event has occurred. In this example of a subject who is suspected to have been exposed to radiation, the P-function predicts the level of exposure. The P-function may be developed in several ways including but not limited to, using techniques based on linear regression and logistic regression. In one technique, the P-function may be expressed as:

$$P/(1 - P) = \exp(\beta_0 + \beta_1 C_1 + \beta_2 C_2 + \beta_3 C_3) \quad \text{Eqn. 2}$$

In this equation, the beta coefficients may be determined by using NHP data, and $C_1$, $C_2$ and $C_3$ are the concentrations of three specific proteins obtained by applying the concentration algorithm to blood samples obtained from the subject. The advantage of using NHP data is that data from humans may not exist or may be difficult to obtain. However, the use of data from humans or other sources is not excluded.

Beta coefficients may be stored in multiple locations such as in the RFID tag, or within the analyzer 200 or in the cloud. This ensures that as more experiments are conducted or other or more data becomes available, better values of the beta coefficients may become available leading to more accurate P values. In one additional feature, the beta coefficients may be associated with a time stamp such that if there is a conflict of which beta coefficients to use, the values with the most recent time-stamp maybe used.

The output of the P-function application is input to the discriminator in box 1520. In this box, the discriminator compares the calculated P-function value to a threshold and outputs a message based on whether the P-function value is at or above the threshold or below the threshold or that the result is indeterminate. For the example of trying to determine the amount radiation exposure, a threshold value corresponding to a 2 Gy exposure may be chosen, based on experiments and other data. Thus, a subject with an exposure level less than 2 Gy may not need immediate treatment whereas a subject with an exposure level of 2 Gy or higher may need immediate treatment. Thus, in box 1525, appropriate messages may be displayed on the display screen 215 of the analyzer 200. Results may also be conveyed by other means such as with an audio message.

Keeping track of the assay development time

The analyzer 200 is configured to keep track of the assay development time or incubation time for the cartridges used with such an analyzer. This is necessary when a certain amount of time is required for the sample to wick or travel to the desired position on the test strips and for the binding reactions to occur. A number of different techniques to keep track of the assay development time are outlined below. In one technique, the cartridge 600 is inserted into the analyzer 200 and kept inside the analyzer until the assay development time has passed. A clock within the analyzer 200, for example, the system clock 357, may be used to keep track of the elapsed time, starting when the cartridge is inserted and advanced into position. In another technique, the start of the assay development time is written to the RFID tag 685 within the cartridge 600. The cartridge may be removed and set aside. The analyzer is left in a powered-on state (or low-powered state (e.g., sleep)) with the cartridge out of the system. The cartridge may be reinserted into the same analyzer (or a different analyzer as detailed below) after some amount of time has passed. Since the same analyzer is being used and the analyzer was kept in a powered-on state (or low-powered state (e.g., sleep)), it is possible to know the elapsed time between the first insertion and the current insertion of the cartridge by reading the time stamp written to the RFID tag and knowing the current time. If the elapsed time is less that the assay development time, a message may be displayed alerting the laboratory technician to eject and reinsert the cartridge at a later time. During the assay development time, the analyzer may also be configured to conduct optical measurements to ensure that the sample is traveling down the test strip. This ensures that the time-stamping process does not start before a sample is present in the cartridge. In addition, if the analyzer detects that there is no flow, an error may be flagged and a message displayed.

In yet another technique, multiple analysis systems are synchronized to the same common time (time synchrony) such as UTC. This may be achieved in multiple ways. In one way, as was illustrated in FIG. 3, each analyzer 600 of a number of analyzers may include a GPS receiver. GPS transmissions include information about the common time so that every analyzer receiving the GPS signal is synchronized to have the same time. In this case, when the cartridge 600 is exposed to the sample, it may be inserted into any analyzer and the start time of the assay development period may be written to the RFID tag as a time stamp. The cartridge may then be removed. At a later time, the cartridge may be inserted into the same or different analyzer. The elapsed time can now be calculated regardless of the analyzer being used, as each analyzer of the number of analyzers is synchronized to the same time.

In a variation of this concept, one analyzer is able to receive the GPS signals including the common time and subsequently broadcast or otherwise make the common time available to other analyzers. In some situations, this may be advantageous as the GPS signal may not be accessible to all the analyzers due to poor satellite signal reception. However, if one analyzer can access the signal and make this time available to other analyzers, then this group of analyzers can be synchronized to the same time. Thus, in this case as above, the first insertion of the cartridge into any analyzer would be accompanied by writing the starting time into the RFID tag on the cartridge. At a subsequent insertion of the cartridge into the same or a different analyzer, the elapsed time may be found based on the common time across these analyzers. With these and the other techniques described above, one or a few analyzers can be utilized as an "initial" analyzer, and then some of other analyzers can be used for the measuring and analysis.

Stability of the developed assays

The developed assays remain stable over a long period of time such as, but not limited to, 5 years or more. After the assay has developed, the test cartridge may be stored and subsequently read by any analyzer of the type described in FIG. 2. This stability results from the nature of the binding that occurs at the target capture zones within the test strips and the long-term photo-stability of the upconverting phosphor reporters. Additional benefits may be realized as the information about the subject may be stored within the RFID tag within the cartridge. One benefit is that the RFID tag may be uniquely associated with a subject minimizing or removing errors due to incorrect association of an analysis result to the subject.

Update of information in the cartridge or in the system

Again, the analyzer 200 and the cartridge 600 can each include storage (e.g., the analyzer can include the memory 310, the cartridge 600 can include the RFID tag 685, etc.) where information such as, but not limited to, the coefficients of the Eqn. 1 may be stored. In one configuration, the information may be updated through various mechanisms including, but not limited to, Wi-Fi networks and USB thumb drives. In one example of how this may be accomplished, when the analyzer is turned on to perform a test, it may automatically connect to a secure website to see if an update is available. If so, it may provide an option to the user to download and accept the update. The update may include information that may be utilized by the analyzer, the cartridge, or both. In one example, as described above, new beta parameters of Eqn. 2 may be downloaded to the analyzer. New values for these beta parameters may become available due to analysis carried out on additional data that was not available when the biological sample-analyzing system 100 was released to the marketplace.

In a variation, the information needed by the analyzer may be stored in the RFID tag of the cartridge. This information may be written into the RFID tag at the time of manufacture of the cartridge. The information may include, but may not be limited to, coefficients, parameters, and equations. As an example, a cartridge may be developed that uses only two proteins to determine the presence or absence of a specific condition in a patient. Thus, a new equation similar to Eqn. 2 may be necessary but with only two variables (e.g., for concentration values). This new equation may be stored within the RFID tag. Upon insertion of this specific cartridge, the information in the tag is designed to be read by the analyzer and this equation stored in the RFID tag would be utilized. Furthermore, the RFID tag may contain the information that may be used by the analyzer to display an appropriate message. Continuing with the example of the condition needing only two proteins, the RFID tag may contain the name of the specific condition that is being tested. This name may be read by the analyzer and used as part of the display message.

Thus, it can be seen that these and other similar techniques provide flexibility in the type and number of molecules that can be detected. Other decisions about the presence and absence of specific conditions based on the presence and absence of specific target molecules may also be made by the analyzer.

Error checking

As indicated in the sections above, error checking may be done throughout the operation of the analyzer. In addition to the error checking mentioned earlier, other error checking algorithms may be implemented. A few examples are provided below. In one example, in regard to the process related to obtaining the intensity readouts, the RFID tag contained in the cartridge is configured to inform the analyzer of the expected location of assay target and control sites. If the analyzer does not find the target or control lines in the expected locations, an error may be flagged and a failure message may be displayed. The target and control sites not being in their expected location may be caused by a damaged or improperly handled cartridge. The failure of the cartridge may also be written into the RFID tag. In another example, the RFID tag may contain information about the expected physiological range of protein concentrations for the proteins being analyzed. If the analyzer determines that concentration of any protein that is being analyzed is outside the human physiological range, an error may be flagged and a message may be displayed. In addition, this information may be written into the RFID tag. In yet another example, if the analyzer detects internal communications errors, motor faults, laser turn-on, laser overcurrent, low battery, 3.3 and 5V power rail voltages out of specification, laser over-temperature, laser optical power monitor signals out of range, optical detector signals out of range, or an unacceptable degradation in laser power over time, errors may be flagged. Any error detected may be configured to display an appropriate message or an alert.

Alternate configurations of the biological sample-analyzing system

An up-converting phosphor based reporter can be used to determine the concentration of target molecules as described herein. However, the concepts described herein may be applied to other configurations including those that do not use the phosphor-reporter molecules. In one example of an alternate configuration for the phosphor reporter-based system, it was described earlier that the analyzer 200 of FIG. 2 utilizes the VCSEL array as the source of illumination. However, any other diode laser emitting at 980 nm may be used as long as sufficient power is emitted. In another example of an alternate configuration with a different reporter, a diode laser emitting in the 400 nm to 700 nm region may be used to excite reporters made of europium chelate nanoparticles or any other fluorescent label that may be used to tag a reporter antibody. In this case, the optics might benefit from modification; however, the concepts described above are directly applicable.

Other uses of the biological sample-analyzing system

As indicated above, the concepts described may be used for determining the level of radiation exposure. However, the concepts may also be used for various other situations as well. In one example, the system may be used to determine the level of radiation received in the course of treatment of cancer. In another example, the system may be used to determine if a subject (human or non-human) has been exposed to specific biological agents. In yet another example, the system may be used to determine if a subject is infected with a virus or a bacterium. In some other examples, the system may be used to determine the concentration of some specific target molecules in a plant. In general, other areas where the concepts described above may be utilized include, but are not limited to, the area of veterinary medicine, as well as botanical and agricultural sampling. Areas may also include industrial environments such as chemical plants, water management centers, and sewage treatment centers.

In view of the foregoing, a hand-held, point-of-care, up-converting phosphor based analyzer and disposable, single-use cartridges are provided that provide quantitative concentration values for one or multiple target molecules such as, but not limited to, proteins, where the concentration values for the one or multiple target molecules are available at the same time.

The disposable cartridges can include a memory device such as, but not limited to, an RFID tag that can enable the storage of various information such as, but not limited to, identification information that can uniquely identify the cartridge, coefficients that are used in the computations, time stamp values, patient information, equations, and messages. Various types of computations can be carried out including computation of concentration and computation of the probability of radiation exposure.

The information stored in the memory device (e.g., RFID tag) in the cartridge can be different between groups of one to multiple cartridges, where the different groups are designed for different analyses with different biomarkers. In some cases, a manufacturing lot may be a group.

The types of messages contained in the RFID tag of the cartridge include, but are not limited to, informational messages, warning messages, messages that are to be displayed, messages about the name of the target molecule, and molecules that are being measured.

A mechanism to minimize or remove the effect of factors that may cause variations in the quantitative output of the biological sample-analyzing system such as, but not limited to, the variation in the concentration values is included in at least the cartridges. These factors may include, but are not be limited to, variations in incident light intensity, variation in battery power, etc.

A method to minimize or remove the effect of factors that cause variations include a normalization mechanism. The normalization mechanism may be of various types including an area that binds with UPT particles and provides a brightness value that can be used to normalize the readings.

The assay development time may be tracked. One way to keep track of assay development time is to use the time-stamping process and the ability of a group of analyzers to be in time-synchrony with each other.

A group of analyzers can receive a common time signal such as, but not limited to, a GPS signal and, thus, be in time synchrony with each other.

The assay development can be initiated on one analyzer such as with a time stamp, but the assay development can be completed on a different analyzer within a group of analyzers that are in time synchrony with each other.

The developed assay can be analyzed in any analyzer at any time including, but not limited to, after 1 year or after 5 years.

New, updated, or additional information can be input into an analyzer or into a cartridge. The new, updated, or additional information can be input automatically when the analyzer is turned on. The new, updated, or additional information may be used for various purposes such as, but not limited to, updating the beta coefficients in the predictor function that predicts the level of radiation.

Normalizing unknown protein values to known baseline or control values for a given species is also provided. This enables the use of identical classifiers for different species. In particular, the beta coefficients in a regression classifier are identical.

In some embodiments, software used to facilitate algorithms discussed herein can be embodied onto a non-transitory machine-readable medium. A machine-readable medium includes any mechanism that stores information in a form readable by a machine (e.g., a computer). For example, a non-transitory machine-readable medium can include read only memory (ROM); random access memory (RAM); magnetic disk storage media; optical storage media; flash memory devices; Digital Versatile Disc (DVD's), EPROMs, EEPROMs, FLASH memory, magnetic or optical cards, or any type of media suitable for storing electronic instructions.

Note, an application described herein includes but is not limited to software applications, mobile applications, and programs that are part of an operating system application. Some portions of this description are presented in terms of algorithms and symbolic representations of operations on data bits within a computer memory. These algorithmic descriptions and representations are the means used by those skilled in the data processing arts to most effectively convey the substance of their work to others skilled in the art. An algorithm is here, and generally, conceived to be a self-consistent sequence of steps leading to a desired result. The steps are those requiring physical manipulations of physical quantities. Usually, though not necessarily, these quantities take the form of electrical or magnetic signals capable of being stored, transferred, combined, compared, and otherwise manipulated. It has proven convenient at times, principally for reasons of common usage, to refer to these signals as bits, values, elements, symbols, characters, terms, numbers, or the like. These algorithms can be written in a number of different software programming languages such as C, C+, HTTP, Java, or other similar languages. Also, an algorithm can be implemented with lines of code in software, configured logic gates in software, or a combination of both. In an embodiment, the logic consists of electronic circuits that follow the rules of Boolean Logic, software that contain patterns of instructions, or any combination of both.

It should be borne in mind, however, that all of these and similar terms are to be associated with the appropriate physical quantities and are merely convenient labels applied to these quantities. Unless specifically stated otherwise as apparent from the above discussions, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system's registers and memories into other data similarly represented as physical quantities within the computer system memories or registers, or other such information storage, transmission or display devices.

Many functions performed by electronic hardware components can be duplicated by software emulation. Thus, a software program written to accomplish those same functions can emulate the functionality of the hardware components in input-output circuitry. Thus, provided herein are one or more non-transitory machine-readable medium configured to store instructions and data that when executed by one or more processors on the computing device of the foregoing system, causes the computing device to perform the operations outlined as described herein.

While some particular embodiments have been provided herein, and while the particular embodiments have been provided in some detail, it is not the intention for the particular embodiments to limit the scope of the concepts presented herein. Additional adaptations and/or modifications can appear to those of ordinary skill in the art, and, in broader aspects, these adaptations and/or modifications are encompassed as well. Accordingly, departures can be made from the particular embodiments provided herein without departing from the scope of the concepts provided herein.

Further examples of feature combinations taught by the present disclosure are set out in the following numbered clauses:

    1. An analyzing system, comprising:

        a) a test cartridge including

            a port configured to accept a biological sample;
            one or more test strips, each test strip thereof including one or more target capture zones configured to bind one or more targets of the biological sample; and
            a memory device, the memory device is configured to store a first batch of information selected from a group consisting of i) information for identifying one or more assays of the one or more test strips, ii) information for determining a development time for the one or more test strips, iii) information for determining concentration of the one or more targets of the biological sample, iv) information for interpreting assay results for the one or more targets, and v) any combination thereof; and

b) an analyzer including

an opening configured to accept the test cartridge;
a reader/writer device configured to i) read the first batch of information from the memory device of the test cartridge, ii) write a second batch of information to the memory device of the test cartridge including an initial time stamp for the biological sample, and iii) read back to the reader/writer device the initial time stamp for the biological sample at least when a calculation of an elapsed time occurs;
a processor configured to execute i) a stored algorithm configured to calculate the elapsed time from a current time and the initial time stamp read back by the reader/writer device and ii) stored logic configured to ensure that the elapsed time meets or exceeds a development time for the one or more test strips before analyzing the one or more test strips;
one or more sources of electromagnetic radiation configured to excite an up-converting phosphor present in the one or more target capture zones of the one or more test strips; and
a detector configured to detect up-converting phosphor emissions from the one or more target capture zones.

2. The analyzing system of clause 1,
wherein the memory device of the test cartridge is a radio frequency identification ("RFID") tag, the reader/writer device of the analyzer is an RFID tag reader/writer device, and the RFID tag is configured to include the first batch of information.

3. The analyzing system of clause 1,

wherein the test cartridge is a single-use cartridge, and
wherein the first batch of information includes all four of i) the information for identifying the one or more assays of the one or more test strips, ii) the information for determining the development time for the one or more test strips, iii) the information for determining the concentration of the one or more targets of the biological sample, and iv) the information for interpreting assay results for the one or more targets, so that the analyzer does not need to have prior knowledge of the biological sample being tested.

4. The analyzing system of clause 1,

wherein each test strip of the one or more test strips further includes a control target capture zone, and
wherein the processor is further configured to execute a stored normalization algorithm configured to divide an up-converting phosphor emission from the control target capture zone from the up-converting phosphor emissions from the one or more target capture zones for each test strip, thereby removing effects from manufacturing variances in the test strips, manufacturing variances in the one or more sources of electromagnetic radiation, different levels of battery power, and variances in biological sample volumes.

5. The analyzing system of clause 4,
wherein the analyzer is configured to modify its internal calculations to accommodate for the manufacturing variances in the test strips, the manufacturing variances in the one or more sources of electromagnetic radiation, and the different levels of battery power.

6. The analyzing system of clause 1,

wherein the processor is further configured to execute a stored classification algorithm configured to convert the up-converting phosphor emissions from the one or more target capture zones to a determination of whether a test result is i) positive as a condition under the test is present, ii) negative as a condition under the test is absent, or iii) indeterminable, and
wherein the stored logic is configured to ensure the elapsed time meets or exceeds the development time for the one or more test strips before analyzing the one or more test strips in accordance with the first batch of information.

7. The analyzing system of clause 1,

wherein the analyzer further includes a communication interface, and
wherein the processor is further configured to execute a communication module configured to establish a communication channel between the analyzer and one or more additional analyzers to communicate the first batch of information, the second batch of information, or both batches of information related to the test cartridge through the communication interface and over the communication channel with the one or more additional analyzers.

8. The analyzing system of clause 1,

wherein the analyzer further includes a communication interface, and
wherein the processor is further configured to execute a communication module configured to establish a communication channel between the analyzer and a backend database associated with a central server, the central server configured to aggregate and organize epidemiological data and test information from one or more additional analyzers.

9. The analyzing system of clause 1,

wherein the opening of the analyzer includes a lip extension configured to shield measurements from ambient light, and
wherein the analyzer further includes a bandpass filter to prevent sunlight from affecting the measurements of the biological sample from the test cartridge.

10. An analyzing system, comprising:

a) a test cartridge including

a port configured to accept a blood sample;
a microfluidic device including a filter configured to provide a buffer-diluted plasma filtrate from a feed of a buffer-diluted blood sample;
one or more test strips, each test strip thereof including one or more target capture zones that are configured to bind one or more targets in the buffer-diluted plasma filtrate; and
a memory device, the memory device including a first batch of information selected from a group consisting of i) information for identifying one or more assays of the one or more test strips, ii) information for determining a development time for the one or more test strips, iii) information for determining concentration of the one or more targets of the buffer-diluted plasma filtrate, iv) information for interpreting assay results for the one or more targets, and v) any combination thereof; and

b) an analyzer including

an opening configured to accept the test cartridge;
a reader/writer device configured to i) read the first batch of information from the memory device, ii) write a second batch of information to the memory device including an initial time stamp for the buffer-diluted plasma filtrate, and iii) read back the initial time stamp for the buffer-diluted plasma filtrate at least when a calculation of an elapsed time occurs;
a processor configured to execute i) a stored algorithm configured to calculate the elapsed time from a current time and the initial time stamp and ii) stored logic configured to ensure the elapsed time meets or exceeds a development time for the one or more test strips;
one or more sources of electromagnetic radiation configured to excite an up-converting phosphor present in the one or more target capture zones of the one or more test strips; and
a detector configured to detect up-converting phosphor emissions from the one or more target capture zones.

11. The analyzing system of clause 10,
wherein the memory device of the test cartridge is a radio frequency identification ("RFID") tag, the memory device reader of the analyzer is an RFID tag reader/writer device, and the RFID tag is configured to include the first batch of information.

12. The analyzing system of clause 10, further comprising:

a system-check cartridge, where the system-check cartridge includes one or more test strips with a plurality of standard target capture zones with up-converting phosphor concentrations ranging from a low-end standard to a high-end standard of the analyzing system's dynamic range,

wherein analyzing system is configured to ensure up-converting phosphor emissions are present at an expected level for the standard target capture zones and are repeatable within an acceptable level of variance.

13. The analyzing system of clause 10,

wherein each test strip of the one or more test strips further includes a control target capture zone, and wherein the processor is further configured to execute a stored normalization algorithm configured to divide an up-converting phosphor emission from the one or more target capture zones from the up-converting phosphor emissions from the control capture zone for each test strip, thereby removing effects from manufacturing variances in the test strips, manufacturing variances in the one or more sources of electromagnetic radiation, where the one or more sources of electromagnetic radiation are lasers, and different levels of battery power, where a time of use of the cartridge is unpredictable so the stored normalization algorithm compensates for the level of battery power at the time of use.

14. The analyzing system of clause 10,
wherein the microfluidic device includes a mixing-reservoir layer including a mixing reservoir configured to mix the blood sample and a buffer solution to form the feed of the buffer-diluted blood sample.

15. The analyzing system of clause 14,
wherein the microfluidic device further includes a filter layer including the filter and a channeled layer including a number of channels configured to channel the buffer-diluted plasma filtrate to a plasma-and-buffer loading well.

16. The analyzing system of clause 15,
wherein the microfluidic device further includes a splitting layer including a number of holes configured to receive and split the buffer-diluted plasma filtrate into a number of samples equal to the number of holes.

17. The analyzing system of clause 10, wherein an incubation of the blood sample in the test cartridge occurs outside the analyzer after an assignment of the initial time stamp, which allows multiple blood samples, each in their respective test cartridge, to incubate outside the analyzer and increase a throughput of test cartridges per analyzer in the analyzer system.

18. A microfluidic device, comprising:

a mixing-reservoir layer including a mixing reservoir configured to mix a blood sample and a buffer solution to form a blood-and-buffer mixture;
a filter layer including a filter configured to produce a filtrate of plasma and buffer from a feed of the blood-and-buffer mixture;
a channeled layer including a number of channels configured to channel the plasma-and-buffer filtrate to a plasma-and-buffer loading well; and
a splitting layer including a number of holes configured to receive and split the plasma-and-buffer filtrate into a number of samples corresponding to the number of holes.

19. The microfluidic device of clause 18,

wherein the filter is an asymmetric filter having differently sized pores on input and output sides of the filter, where the input side has larger pores than the output side of the filter, and
a mesh layer including a mesh configured to distribute a plasma-and-buffer filtrate over a substantial area of the mesh as well as permit lateral fluid flow while minimizing a dead volume of the plasma-and-buffer filtrate.

20. The microfluidic device of clause 18,
wherein the number of holes in the splitting layer are sized to provide a primary resistance to flow of the plasma-and-buffer filtrate through the microfluidic device.

21. The microfluidic device of clause 18, further comprising:
a number of spacer layers including a first spacer layer and a second spacer layer with the filter layer disposed between the first spacer layer and the second spacer layer, and

a third spacer layer and a fourth spacer layer with the mesh layer disposed between the third spacer layer and the fourth spacer layer.

**Claims**

1. An analyzing system, comprising:

   a) a test cartridge including a port, one or more test strips, and a memory device,

      where the port is configured to accept a biological sample,
      where each test strip thereof is configured to include one or more target capture zones, where each target capture zone is configured to bind one or more targets of the biological sample,
      where the memory device is configured to store a first batch of information that includes at least one of i) information for determining a development time for the one or more test strips, ii) information for determining concentration of the one or more targets of the biological sample, and iii) information for interpreting assay results for the one or more targets; and

   b) an analyzer including an opening, a reader/writer device, a processor, one or more sources of electromagnetic radiation configured to excite, and a detector,

      where the opening is configured to accept the test cartridge,
      where the reader/writer device is configured to i) read the first batch of information from the memory device of the test cartridge, ii) write a second batch of information to the memory device of the test cartridge including an initial time stamp for the biological sample, and iii) read back to the reader/writer device the initial time stamp for the biological sample at least when a calculation of an elapsed time occurs,
      where the processor is configured to execute i) a stored algorithm configured to calculate the elapsed time from a current time and the initial time stamp read back by the reader/writer device and ii) stored logic configured to ensure that the elapsed time meets or exceeds a development time for the one or more test strips before analyzing the one or more test strips,
      where the one or more sources of electromagnetic radiation is configured to excite a reporter particle present in the one or more target capture zones of the one or more test strips,
      where the detector is configured to detect reporter particle emissions from the one or more target capture zones.

2. The analyzing system of claim 1,
   where the reporter particle is at least one of i) an up-converting phosphor reporter particle and ii) a particle made of europium chelate nanoparticles or any other fluorescent label that can be used to tag a reporter antibody.

3. The analyzing system of claim 1,

   wherein each test strip of the one or more test strips further includes a control target capture zone, and
   wherein the processor is further configured to execute a stored normalization algorithm configured to divide an emission from a first reporter particle from the one or more target capture zones of the first reporter particle by emissions from the control target capture zone for each test strip, thereby removing effects from manufacturing variances in the test strips, manufacturing variances in the one or more sources of electromagnetic radiation, different levels of battery power, and variances in biological sample volumes.

4. The analyzing system of claim 1,
   wherein the reporter particle is at least one of:

   an up-converting phosphor based reporter particle, and
   a particle made of europium chelate nanoparticles or any other fluorescent label that can be used to tag a reporter antibody.

5. The analyzing system of claim 1, where the reporter particle is the up-converting phosphor reporter particle.

6. The analyzing system of claim 1, where the reporter particle is the particle made of europium chelate nanoparticles or any other fluorescent label that can be used to tag a reporter antibody.

7. The analyzing system of claim 1, where the memory device of the test cartridge is a radio frequency identification ("RFID") tag, the reader/writer device of the analyzer is an RFID tag reader/writer device, and the RFID tag is configured to include the first batch of information.

8. The analyzing system of claim 1,

wherein the first batch of information includes all four of i) information for identifying the one or more assays of the one or more test strips, ii) the information for determining the development time for the one or more test strips, iii) the information for determining the concentration of the one or more targets of the biological sample, and iv) the information for interpreting assay results for the one or more targets, so that the analyzer does not need to have prior knowledge of the biological sample being tested, and
wherein the stored logic is configured to ensure the elapsed time meets or exceeds the development time for the one or more test strips before analyzing the one or more test strips in accordance with the first batch of information.

9. The analyzing system of claim 1,

wherein the analyzer further includes a communication interface, and
wherein the processor is further configured to cooperate with a communication module configured to establish a communication channel between the analyzer and one or more additional analyzers to communicate the first batch of information related to the test cartridge through the communication interface and over the communication channel with the one or more additional analyzers.

10. The analyzing system of claim 1,

wherein the opening of the analyzer includes a lip extension configured to shield measurements from ambient light, and
wherein the analyzer further includes a bandpass filter to prevent sunlight from affecting the measurements of the biological sample from the test cartridge.

11. A method for an analyzing system, comprising:

a) configuring a test cartridge to include a port, one or more test strips, and a memory device,

configuring the port to accept a biological sample,
configuring each test strip thereof to include one or more target capture zones, configuring each target capture zone to bind one or more targets of the biological sample,
configuring the memory device to store a first batch of information that includes at least one of i) information for determining a development time for the one or more test strips, ii) information for determining concentration of the one or more targets of the biological sample, and iii) information for interpreting assay results for the one or more targets; and

b) configuring an analyzer to include an opening, a reader/writer device, a processor, one or more sources of electromagnetic radiation configured to excite, and a detector,

configuring the opening to accept the test cartridge,
configuring the reader/writer device to i) read the first batch of information from the memory device of the test cartridge, ii) write a second batch of information to the memory device of the test cartridge including an initial time stamp for the biological sample, and iii) read back to the reader/writer device the initial time stamp for the biological sample at least when a calculation of an elapsed time occurs,
configuring the processor to execute i) a stored algorithm configured to calculate the elapsed time from a current time and the initial time stamp read back by the reader/writer device and ii) stored logic configured to ensure that the elapsed time meets or exceeds a development time for the one or more test strips before analyzing the one or more test strips,
configuring the one or more sources of electromagnetic radiation to excite a reporter particle present in the one or more target capture zones of the one or more test strips,
configuring the detector to detect reporter particle emissions from the one or more target capture zones.

**12.** The method for the analyzing system of claim 11, further comprising:
configuring the reporter particle to be at least one of i) an up-converting phosphor reporter particle and ii) a particle made of europium chelate nanoparticles or any other fluorescent label that can be used to tag a reporter antibody.

**13.** The method for the analyzing system of claim 11, further comprising:

configuring the each test strip thereof to include at least several target capture zones, and
configuring the detector to detect the reporter particle emissions from the several target capture zones.

**14.** The method for the analyzing system of claim 11, further comprising:
configuring the memory device of the test cartridge to be a radio frequency identification ("RFID") tag, the reader/writer device of the analyzer to be an RFID tag reader/writer device, and the RFID tag to include the first batch of information.

**15.** The method for the analyzing system of claim 11, further comprising:

wherein the first batch of information includes all four of i) information for identifying the one or more assays of the one or more test strips, ii) the information for determining the development time for the one or more test strips, iii) the information for determining the concentration of the one or more targets of the biological sample, and iv) the information for interpreting assay results for the one or more targets, so that the analyzer does not need to have prior knowledge of the biological sample being tested,
and
configuring the stored logic to ensure the elapsed time meets or exceeds the development time for the one or more test strips before analyzing the one or more test strips in accordance with the first batch of information.

FIG. 1

**FIG. 2**

**FIG. 3**

EP 4 129 485 A1

EP 4 129 485 A1

| VCSEL Illumination Source | → Collimating Lens → | Turning Mirrors → | Dichroic Filter | ⇒ | Power Monitor |

**FIG. 4A**

**FIG. 4B**

900

570

600

700

800

565

**FIG. 5**

600

570

565A

680

565B

685

**FIG. 6**

EP 4 129 485 A1

700

50.73 mm

28.00 mm

21.75 mm

17.70 mm

715    727    727

**FIG. 7A**

**FIG. 7B**

**FIG. 8**

FIG. 9

1200

Collect raw sample — 1202

Insert sample collector into cartridge — 1205

Force the raw sample and buffer out of the sample collector — 1210

Mixer Separator splitter — 1215

Add reporter particles to sample — 1220

Allow sample and reporters to flow over test strips — 1225

Proteins in sample bind to probe molecules on strips — 1230

Excite the strips with light and detect the up-converted light. Convert into concentration — 1235

1300

Scan the assay strips
(2xN array) —— 1305

Subtract
Background —— 1310

Find peaks —— 1315

Normalize —— 1317

Error Check —— 1320

Apply standard
curves —— 1325

Calculate
concentration —— 1327

**FIG. 10**

FIG. 11

1500

```
┌─────────────────────────┐
│      Concentration      │ ── 1505
│         values          │
└─────────────────────────┘
            │
            ▼
┌─────────────────────────┐
│   Log transformation    │ ── 1510
│            &            │
│      Normalization      │
└─────────────────────────┘
            │
            ▼
┌─────────────────────────┐
│       P function        │ ── 1515
└─────────────────────────┘
            │
            ▼
┌─────────────────────────┐
│      Discriminator      │ ── 1520
└─────────────────────────┘
            │
            ▼
┌─────────────────────────┐
│         Result          │ ── 1525
└─────────────────────────┘
```

**FIG. 12**

Europäisches
Patentamt

European
Patent Office

Office européen
des brevets

# EUROPEAN SEARCH REPORT

**Application Number**

EP 22 19 1524

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/009122 A1 (WHELAN JAMES P [US] ET AL) 13 January 2005 (2005-01-13) | 1-9, 11-15 | INV. B01L99/00 G01N33/48 |
| Y | * 0018-0020,0022,0023,0027,0031,0034 * * figures 1, 2, 3A * | 10 | G01N33/487 G01N33/49 |
| Y | US 2013/162981 A1 (EMERIC PIERRE [US] ET AL) 27 June 2013 (2013-06-27) | 10 | B01L3/00 |
| A | * paragraphs [0059], [0060] – [0062], [0065] – [0067], [0073], [0075], [0076], [0081] – paragraphs [0083], [0092], [0093], [0095] – [0098] * * figures 1B, 2, 3A-3D, 6, 7, 14, 15A-15C, 16A-16C * | 1-9, 11-15 | |
| Y | US 8 883 489 B2 (PANG ALAN [GB]; JOWETT GORDON [GB] ET AL.) 11 November 2014 (2014-11-11) | 10 | |
| A | * column 5, lines 10-12, 34-36 * * column 5, line 56 – column 6, line 4 * * column 6, line 29 – paragraph 33 * * column 7, line 15 – line 30 * * column 8, line 41 – line 52 * * figures 2, 4, 6 * | 1-9, 11-15 | |
| A | US 2013/230844 A1 (EGAN RICHARD L [US] ET AL) 5 September 2013 (2013-09-05) * paragraphs [0050], [0051], [0054], [0058], [0064] – paragraphs [0066], [0069], [0078] – [0080], [0092], [0093] * * figures 1A-1B, 2A-2B, 4, 5, 6, 8, 9A-9B, 11 * | 1-15 | **TECHNICAL FIELDS SEARCHED (IPC)** B01L G16H |
| A | US 2016/169882 A1 (SNIDER JAMES V [US] ET AL) 16 June 2016 (2016-06-16) * the whole document * | 1-15 | |

-/--

The present search report has been drawn up for all claims

| Place of search | Date of completion of the search | Examiner |
|---|---|---|
| The Hague | 11 October 2022 | Bischoff, Laura |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

**EP 22 19 1524**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2015/293085 A1 (ANDERBERG JOSEPH [US] ET AL) 15 October 2015 (2015-10-15) * the whole document * | 1-15 | |
| A | WO 2014/126918 A1 (CHARM SCIENCES INC [US]) 21 August 2014 (2014-08-21) * the whole document * | 1-15 | |

TECHNICAL FIELDS
SEARCHED    (IPC)

The present search report has been drawn up for all claims

| Place of search | Date of completion of the search | Examiner |
|---|---|---|
| The Hague | 11 October 2022 | Bischoff, Laura |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 19 1524

This annex lists the patent family members relating to the patent documents cited in the above-mentioned European search report.
The members are as contained in the European Patent Office EDP file on
The European Patent Office is in no way liable for these particulars which are merely given for the purpose of information.

11-10-2022

| Patent document cited in search report | | Publication date | Patent family member(s) | | Publication date |
|---|---|---|---|---|---|
| US 2005009122 | A1 | 13-01-2005 | NONE | | |
| US 2013162981 | A1 | 27-06-2013 | US | 2013162981 A1 | 27-06-2013 |
| | | | US | 2016041104 A1 | 11-02-2016 |
| | | | WO | 2013096801 A1 | 27-06-2013 |
| US 8883489 | B2 | 11-11-2014 | AU | 2011294873 A1 | 07-03-2013 |
| | | | CA | 2812966 A1 | 01-03-2012 |
| | | | CN | 103079704 A | 01-05-2013 |
| | | | CN | 105759027 A | 13-07-2016 |
| | | | EP | 2608885 A1 | 03-07-2013 |
| | | | EP | 3100787 A1 | 07-12-2016 |
| | | | EP | 3527289 A1 | 21-08-2019 |
| | | | ES | 2754451 T3 | 17-04-2020 |
| | | | GB | 2483077 A | 29-02-2012 |
| | | | JP | 2013536431 A | 19-09-2013 |
| | | | NZ | 607045 A | 31-10-2014 |
| | | | US | 2013157381 A1 | 20-06-2013 |
| | | | US | 2015030504 A1 | 29-01-2015 |
| | | | US | 2018017548 A1 | 18-01-2018 |
| | | | US | 2019302104 A1 | 03-10-2019 |
| | | | WO | 2012025729 A1 | 01-03-2012 |
| US 2013230844 | A1 | 05-09-2013 | US | 2013230844 A1 | 05-09-2013 |
| | | | US | 2016054316 A1 | 25-02-2016 |
| | | | US | 2019271695 A1 | 05-09-2019 |
| | | | US | 2021389316 A1 | 16-12-2021 |
| | | | WO | 2013131057 A1 | 06-09-2013 |
| US 2016169882 | A1 | 16-06-2016 | US | 2016169882 A1 | 16-06-2016 |
| | | | US | 2019013100 A1 | 10-01-2019 |
| | | | US | 2020035357 A1 | 30-01-2020 |
| US 2015293085 | A1 | 15-10-2015 | CN | 105051542 A | 11-11-2015 |
| | | | EP | 2923204 A1 | 30-09-2015 |
| | | | HK | 1215970 A1 | 30-09-2016 |
| | | | US | 2015293085 A1 | 15-10-2015 |
| | | | US | 2017234867 A1 | 17-08-2017 |
| | | | WO | 2014070935 A1 | 08-05-2014 |
| WO 2014126918 | A1 | 21-08-2014 | US | 2017184585 A1 | 29-06-2017 |
| | | | WO | 2014126918 A1 | 21-08-2014 |

EPO FORM P0459

For more details about this annex : see Official Journal of the European Patent Office, No. 12/82

**EP 4 129 485 A1**

**REFERENCES CITED IN THE DESCRIPTION**

**Patent documents cited in the description**

- US 62373755
- US 62400176
- US 62400184